(19) Europäisches Patentamt
European Patent Office
Office européen des brevets

(11) EP 4 265 593 A1

(12) **EUROPEAN PATENT APPLICATION**
published in accordance with Art. 153(4) EPC

(43) Date of publication:
**25.10.2023 Bulletin 2023/43**

(21) Application number: **21910778.6**

(22) Date of filing: **21.12.2021**

(51) International Patent Classification (IPC):
***C07C 69/76*** *(2006.01)* ***C07C 69/78*** *(2006.01)*
***C07C 69/92*** *(2006.01)* ***C08F 10/06*** *(2006.01)*

(52) Cooperative Patent Classification (CPC):
**C07C 69/76; C07C 69/78; C07C 69/92;** C08F 10/06

(86) International application number:
**PCT/JP2021/047303**

(87) International publication number:
**WO 2022/138635 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.12.2020 JP 2020211716**

(71) Applicant: **Mitsui Chemicals, Inc.**
**Tokyo 104-0028 (JP)**

(72) Inventors:
- **YANO Takaaki**
  **Sodegaura-shi, Chiba 299-0265 (JP)**
- **YAMADA Wataru**
  **Sodegaura-shi, Chiba 299-0265 (JP)**
- **TAKANO Shotaro**
  **Sodegaura-shi, Chiba 299-0265 (JP)**
- **KIMURA Takashi**
  **Kuga-gun, Yamaguchi 740-0061 (JP)**
- **ISOGAI Makoto**
  **Kuga-gun, Yamaguchi 740-0061 (JP)**
- **NAKANO Takashi**
  **Sodegaura-shi, Chiba 299-0265 (JP)**
- **MOORTHI Sunil Krzysztof**
  **Sodegaura-shi, Chiba 299-0265 (JP)**

(74) Representative: **J A Kemp LLP**
**80 Turnmill Street**
**London EC1M 5QU (GB)**

(54) **ESTER COMPOUND**

(57) [Problem] To provide an internal donor component capable of producing, with high productivity, a propylene polymer having extremely high stereoregularity when used mainly as a solid titanium catalyst component.

[Solution] A polyol ester compound having a specific polycyclic structure represented by formula (1). This ester compound is characterized in that sites including a C-$R^3$ structure and a C-$R^4$ structure satisfy a specific relationship.

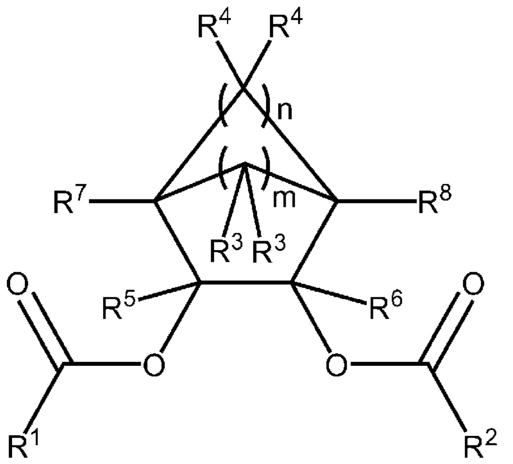

(1)

EP 4 265 593 A1

## Description

### Technical Field

The present invention relates to a novel ester compound.

### Background Art

As conventional techniques relating to ester compounds, there are many disclosures relating to applications as additives for resin additives, cosmetics and external preparations for the skin, microbicidal compositions, antioxidants, and chelators, for example. As one of such applications, there is known an embodiment for use in a Mg compound-supported titanium catalyst for use in olefin polymerization.

A catalyst for olefin polymerization is one of techniques greatly developed up to the present, which was triggered by the discovery of so-called Ziegler-Natta catalysts, for which Ziegler reported in 1953 that ethylene was polymerized even at low pressures by use of a combination of titanium tetrachloride and an organoaluminum compound and Natta subsequently reported the first propylene polymerization by use of a combination of titanium trichloride and a halogen-containing organoaluminum compound. Meanwhile, it has been found that catalysts containing titanium tetrachloride, a magnesium compound, and a Lewis base, which are referred to as third generation catalysts, can achieve both high polymerization activity (high productivity) and high stereoregularity in propylene polymerization. This provided one opportunity to allow propylene polymers (polypropylene) to spread around the world.

A Lewis base (hereinafter, also referred to as an "internal donor"), one of major components of the above third generation catalyst component (hereinafter, also referred to as a "solid titanium catalyst component"), was found to greatly affect catalyst performance, and various Lewis bases have been developed so far.

As Lewis bases for use in Ziegler-Natta catalysts, ethyl benzoate, phthalic esters, 1,3-diketone (Patent Literature 1), malonic ester (Patent Literature 2), succinic ester (Patent Literature 3), 2,4-pentanediol diester (Patent Literature 4), naphthalenediol diester (Patent Literature 5), and catechol diester (Patent Literature 6), for example, have been reported. Mainly enterprises vigorously make research and development in this field even today.

With respect to elementary reactions for synthesis of various ester compound, a large number of approaches have been disclosed (e.g., Patent Literatures 7 to 10 and Non-Patent Literatures 1 to 19).

### Citation List

#### Patent Literature

Patent Literature 1: JP 2005-226076A
Patent Literature 2: JP 2000-516987A
Patent Literature 3: JP 2002-542347A
Patent Literature 4: JP 2005-517746A
Patent Literature 5: JP 2011-529888A
Patent Literature 6: JP 2014-500390A
Patent Literature 7: JP 2008-247796A
Patent Literature 8: WO2008/062553
Patent Literature 9: US 2018/0149973A1
Patent Literature 10: US 2002/0162991A1


#### Non-Patent Literature

Non-Patent Literature 1: Journal of Organic Chemistry, 1969, 34, 3579-3582
Non-Patent Literature 2: Journal of Organic Chemistry, 1971, 36, 3979-3987
Non-Patent Literature 3: Organic Letters, 2004, 6, 1589-1592
Non-Patent Literature 4: Journal of the American Chemical Society, 1957, 79, 2822-2824
Non-Patent Literature 5: Organic Synthesis 1991, 70, 47-53
Non-Patent Literature 6: Organic Synthesis 1997, 75, 153-160
Non-Patent Literature 7: Catalysis Letters 2012, 142, 124-130

Non-Patent Literature 8: Organic Synthesis 1997, 74, 91-100
Non-Patent Literature 9: The Fourth Series of Experimental Chemistry, vol. 20, Synthesis of Organic Compound II, Alcohols and amines, p.39
Non-Patent Literature 10: Journal of Organic Chemistry 1959, 24, 54-55
Non-Patent Literature 11: Angewandte Chemie International Edition, 1978, 17, 522-524
Non-Patent Literature 12: Bulletin of the Chemical Society of Japan, 1967, 40, 2380-2382
Non-Patent Literature 13: Organic Synthesis, 1952, 32, 41
Non-Patent Literature 14: Macromolecules, 2017, 50, 580-586
Non-Patent Literature 15: Journal of Organic Chemistry, 1980, 45, 2301-2304
Non-Patent Literature 16: Journal of Organic Chemistry, 2009, 74, 405-407
Non-Patent Literature 17: Journal of Organic Chemistry, 1988, 53, 2120-2122
Non-Patent Literature 18: Journal of Organic Chemistry, 1963, 28, 2572-2577
Non-Patent Literature 19: European Journal of Organic Chemistry, 2017, 24, 3501-3504

## Summary of Invention

### Technical Problem

Propylene polymers, while having heat resistance and rigidity similar to those of general-purpose engineering plastics, have an advantage of generating a smaller amount of toxic gas even when combustion-treated, because of being constituted substantially only by carbon and hydrogen.

With recent advances in molding techniques, use of a propylene polymer having higher stereoregularity than before has a possibility of developing higher physical properties (such as rigidity and heat resistance). For this reason, the market has required propylene polymers having higher stereoregularity. From the viewpoint of resource saving and environmental protection, methods for producing a propylene polymer at a high productivity also have been required.

It is thus an object of the present invention to provide an internal donor component suitable for a solid titanium catalyst component capable of producing a propylene polymer having extremely high stereoregularity at a high productivity (high activity), when used mainly for solid titanium catalyst component.

### Solution to Problem

As a result of diligent study to solve the above problems, the present inventors have found that an ester compound having a specific cyclic structure is suitable as, for example, a Lewis base for a solid titanium catalyst component, and completed the present invention. The present invention relates to, for example, the following [1] to [4].

[1] A cyclic multiple-ester-group-containing compound (A) represented by the following formula (1):

[Formula 1]

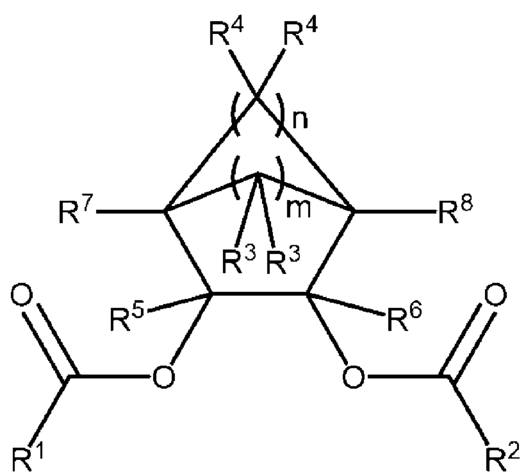

(1)

wherein m and n are each independently an integer of 1 to 5, with a relationship of m+n ≥ 4 being satisfied;

$R^1$ and $R^2$ are each independently a substituted or unsubstituted hydrocarbon group having 1 to 20 carbon atoms; a plurality of $R^3$, a plurality of $R^4$, and $R^5$ to $R^8$ are each independently a group selected from a hydrogen atom, a substituted or unsubstituted hydrocarbon group having 1 to 20 carbon atoms, or a halogen atom; a hydrogen atom, a carbon atom, or both, of $R^1$ to $R^8$ are optionally replaced by at least one atom selected from the group consisting of a nitrogen atom, an oxygen atom, a phosphorus atom, a halogen atom, and a silicon atom; while $R^3$ to $R^8$ are in a mutually independent relationship, adjacent $R^3$ groups are optionally directly bonded to one another to form a multiple bond; adjacent $R^4$ groups are optionally directly bonded to one another to form a multiple bond; and a plurality of $R^3$ bonded to the same carbon, and a plurality of $R^4$ bonded to the same carbon, are optionally bonded to one another to form a cyclic structure.

[2] The ester compound (A) according to [1], wherein m is 2 or more, and n is 2 or more.
[3] The ester compound (A) according to [1], wherein $R^1$ and $R^2$ are each independently a substituted or unsubstituted alkyl group, a substituted or unsubstituted alkenyl group, a substituted or unsubstituted cycloalkyl group, a substituted or unsubstituted aryl group, or a substituted or unsubstituted heteroaryl group.
[4] The ester compound (A) according to [1], wherein $R^3$ to $R^8$ are each independently a group selected from a hydrogen atom, a substituted or unsubstituted alkyl group, a substituted or unsubstituted alkenyl group, a substituted or unsubstituted cycloalkyl group, a substituted or unsubstituted cycloalkenyl group, a substituted or unsubstituted alkoxy group, a substituted or unsubstituted alkenyloxy group, a substituted or unsubstituted cycloalkyloxy group, a substituted or unsubstituted cycloalkenyloxy group, a substituted or unsubstituted aryl group, a substituted or unsubstituted aryloxy group, a substituted or unsubstituted heteroaryl group, or a substituted or unsubstituted heteroaryloxy group.

## Advantageous Effects of Invention

The ester compound of the present invention can be used in resin additives, cosmetics and external preparations for the skin, microbicidal compositions, antioxidants, chelators, and Ziegler-Natta catalysts, for example.

## Description of Embodiments

Hereinafter, an ester compound according to the present invention will now be described in detail.
The ester compound according to the present invention (hereinafter, also referred to as the "ester compound (A)") is represented by the following general formula (1):

[Formula 2]

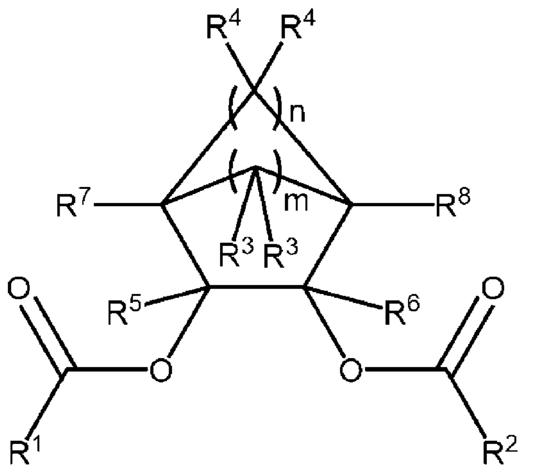

(1)

wherein, $R^1$ and $R^2$ are each independently a substituted or unsubstituted hydrocarbon group having 1 to 20 carbon atoms. A hydrogen atom, a carbon atom, or both, of $R^1$ and $R^2$ may be replaced by at least one atom selected from the group consisting of a nitrogen atom, an oxygen atom, a phosphorus atom, a halogen atom, and a silicon atom (hereinafter sometimes referred to as hetero atoms). The group of heteroatoms is preferably a group consisting of a nitrogen atom, an oxygen atom, a phosphorus atom, and a silicon atom, more preferably a group consisting of a nitrogen atom, an oxygen atom, and a silicon atom, and even more preferably a group consisting of an oxygen atom and a silicon atom.

The heteroatom-containing substituent is, for example, a heteroatom-containing hydrocarbon group, a heteroatom-containing aryl group is a preferable example, and an oxygen-containing aryl group is a particularly preferable example. Examples of the heteroatom-containing aryl group include those with a basic skeleton having a structure in which the aryl structure itself contains a heteroatom, such as a pyrrole ring or a pyran ring, and those in which a substituent such as a heteroatom-containing hydrocarbon group, e.g., an alkoxy group, is bonded to a benzene ring.

$R^1$ and $R^2$ are each preferably a hydrocarbon group having 2 to 20 carbon atoms, and the lower limit of the number of carbon atoms is more preferably 4, and even more preferably 6. A more detailed structure will be described below.

In the present invention, the term "atom" as in, for example, a halogen atom or a hydrogen atom in the description of a substituent may refer to, as a matter of course, aspects with a bond such as "H-" or "Cl-" when expressed in a structural formula.

Examples of the hydrocarbon group can include substituted or unsubstituted alkyl groups, substituted or unsubstituted cycloalkyl groups, substituted or unsubstituted alkenyl groups, substituted or unsubstituted alkynyl groups, and substituted or unsubstituted aryl groups.

Examples of the hydrocarbon group containing a heteroatom (hereinafter sometimes referred to as a hetero atom-containing hydrocarbon group) can include substituted or unsubstituted hetero atom-containing alkyl groups and substituted or unsubstituted heteroaryl groups.

Examples of the hydrocarbon group and the hetero atom-containing hydrocarbon group include alkyl groups, cycloalkyl groups, alkenyl groups, alkynyl groups, aryl groups, hetero atom-containing alkyl groups, and heteroaryl groups. These groups preferably have 1 to 20 carbon atoms. The lower limit is preferably 2, more preferably 3, and particularly preferably 4. However, a preferable lower limit in the case of an aryl group is 6. On the other hand, the upper limit is preferably 18, more preferably 15, even more preferably 10, and particularly preferably 6. A heteroaryl group preferably has one or more 5-membered ring structures, more preferably has one or more 5 to 7-membered ring structures, and further preferably has one or more 5-membered ring or 6-membered ring structure.

Preferable $R^1$ to $R^2$ are each independently a group selected from a substituted or unsubstituted alkyl group having 4 to 20 carbon atoms, a substituted or unsubstituted cycloalkyl group having 4 to 20 carbon atoms, a substituted or unsubstituted alkenyl group having 4 to 20 carbon atoms, a substituted or unsubstituted alkynyl group having 4 to 20 carbon atoms, a substituted or unsubstituted aryl group having 6 to 20 carbon atoms, a substituted or unsubstituted hetero atom-containing alkyl group having 4 to 20 carbon atoms, or a substituted or unsubstituted heteroaryl group having 4 to 20 carbon atoms.

More preferable $R^1$ and $R^2$ are each independently a group selected from a substituted or unsubstituted alkyl group having 4 to 10 carbon atoms, a substituted or unsubstituted aryl group having 6 to 15 carbon atoms, a substituted or unsubstituted hetero atom-containing alkyl group having 4 to 10 carbon atoms, or a substituted or unsubstituted heteroaryl group having 4 to 15 carbon atoms.

Particularly preferable $R^1$ and $R^2$ are each independently a substituted or unsubstituted aryl group having 6 to 10 carbon atoms and a substituted or unsubstituted heteroaryl group having 4 to 10 carbon atoms, and a group selected from substituted or unsubstituted aryl groups having 6 to 10 carbon atoms is especially preferable.

$R^1$ and $R^2$ of the present invention may be bonded to $R^3$ to $R^8$, which will be described below, to form a monocyclic structure or a polycyclic structure. $R^1$ and $R^2$ may be bonded to one another to form a cyclic structure.

<$R^3$ to $R^8$>

In the formula (1), for example, $R^3$ to $R^8$ are each independently selected from a hydrogen atom, a halogen atom, a hydrocarbon group, or a hetero atom-containing hydrocarbon group.

A preferable example of the group of heteroatoms is a group consisting of a nitrogen atom, an oxygen atom, a phosphorus atom, a halogen atom, and a silicon atom. The group of heteroatoms is more preferably a group consisting of a nitrogen atom, an oxygen atom, a phosphorus atom, and a silicon atom, even more preferably a group consisting of a nitrogen atom, an oxygen atom, and a silicon atom, and particularly preferably a group consisting of an oxygen atom and a silicon atom. The oxygen atom-containing substituent is preferably an ether type (a substituent containing a C-O-C type structure), and, preferably, a structure containing an oxygen double bond is avoided.

Examples of the hydrocarbon group can include substituted or unsubstituted alkyl groups, substituted or unsubstituted cycloalkyl groups, substituted or unsubstituted alkenyl groups, substituted or unsubstituted alkynyl groups, and substituted or unsubstituted aryl groups.

Examples of the hetero atom-containing hydrocarbon group can include substituted or unsubstituted hetero atom-containing alkyl groups, and hetero atom-containing aryl groups such as substituted or unsubstituted heteroaryl groups. Examples of the heteroatom-containing aryl group include those with a basic skeleton having a structure in which the aryl structure itself contains a heteroatom, such as a pyrrole ring or a pyran ring, and those in which a substituent such as a heteroatom-containing hydrocarbon group, e.g., an alkoxy group, is bonded to a benzene ring.

Examples of the hydrocarbon group and the hetero atom-containing hydrocarbon group include alkyl groups, cycloalkyl groups, alkenyl groups, alkynyl groups, aryl groups, hetero atom-containing alkyl groups, and heteroaryl groups. These groups preferably have 1 to 20 carbon atoms. The lower limit is preferably 2, and more preferably 3. However, a preferable lower limit in the case of an aryl group is 6. On the other hand, the upper limit is preferably 18, more preferably 15, even more preferably 10, and particularly preferably 6. The heteroaryl group preferably has one or more 5-membered ring structures, more preferably has one or more 5 to 7-membered ring structures, and further preferably has one or more 5-membered ring or 6-membered ring structure.

Preferably, at least one of $R^3$ to $R^8$ is a substituent of the above preferable embodiments, and, more preferably, all are substituents of the above preferable embodiments.

At least one substituent of $R^3$ to $R^8$ may be preferably a substituent other than hydrogen. Moreover, two or more substituents may preferably be substituents other than hydrogen. In such a case, two or more kinds of substituents may be concomitantly present, or all substituents may be of a single kind. Moreover, one or more carbon atoms that form the cyclic structure may preferably be quaternary carbon. More preferably, $R^7$ and/or $R^8$ may be preferably a substituent other than hydrogen. Particularly preferably, $R^7$ and/or $R^8$ is a hydrocarbon group or a heteroatom-containing hydrocarbon group, and in particular a hydrocarbon group. In such an embodiment, when the ester compound of the present invention is used as a component of a catalyst for olefin polymerization, the performance balance may be improved. Specific examples of the performance include reaction control performance such as activity, stereospecificity, and molecular weight controllability.

A plurality of $R^3$ may be present. In such a case, while $R^3$ groups bonded to different carbon atoms are in a mutually independent relationship, $R^3$ groups bonded to adjacent carbon atoms may be directly bonded to one another to form a multiple bond. Here, being in an independent relationship means that a plurality of $R^3$ groups can be clearly distinguished from each other in terms of structural formula, and, specifically, refers to, for example, a structure in which a plurality of $R^3$ groups that are bonded to different carbon atoms are not bonded to one another to form a ring structure having three or more ring members.

A plurality of $R^4$ may be present. In such a case, while $R^4$ groups bonded to different carbon atoms are in a mutually independent relationship, $R^4$ groups bonded to adjacent carbon atoms may be directly bonded to one another to form a multiple bond. Here, being in an independent relationship is as defined for $R^3$, and means that a plurality of $R^4$ groups can be clearly distinguished from each other in terms of structural formula, and, specifically, refers to, for example, a structure in which a plurality of $R^4$ groups are not bonded to one another to form a ring structure having three or more ring members. $R^3$ groups bonded to the same carbon may be bonded to one another to form a monocyclic or polycyclic ring. Also, $R^4$ groups bonded to the same carbon may be bonded to one another to form a monocyclic or polycyclic ring.

The carbon chain structure including the C-$R^3$ structure and the C-$R^4$ structure of the formula (1) may be a structure composed of any of a single bond, a double bond, and a triple bond, and is preferably composed mainly of a single bond. A heteroatom may be present between the carbon chain bonds. Examples of such chain structures include (divalent) structural formulae as shown below:

[Formula 3]

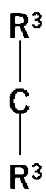
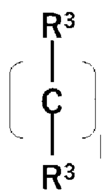
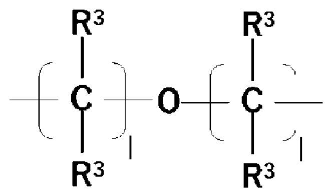
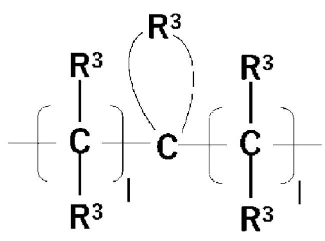

(l is a natural number.)

One substituent each of $R^5$-$R^8$ is present. While $R^5$ to $R^8$, as with $R^3$ and $R^4$, are in an independent relationship, substituents ($R^3$ to $R^8$) bonded to adjacent carbons may be directly bonded to one another so that $R^5$ to $R^8$ may form a multiple bond.

In the formula (1), m and n are each independently selected from an integer of 1 to 5, with a relationship of $m+n \geq 4$ being satisfied.

m and n are numerical values relating to the size and balance of the cyclic structure. A preferable lower limit of m and n is 2. Moreover, m and n are both 2 or more in a preferable embodiment. The upper limit of m and n is 5, and a preferable upper limit is 4. The numerical values of m and n may be the same or different.

Examples of the halogen atom include fluorine, chlorine, bromine, and iodine.

Examples of the substituted or unsubstituted alkyl group include a methyl group, an ethyl group, a n-propyl group, an isopropyl group, a n-butyl group, an isobutyl group, a sec-butyl group, a tert-butyl group, a neopentyl group, a n-hexyl group, a thexyl group, a cumyl group, and a trityl group.

Examples of the substituted or unsubstituted alkenyl group include a vinyl group, an allyl group, a propenyl group, an isopropenyl group, a butenyl group, an isobutenyl group, a pentenyl group, and a hexenyl group.

Examples of the substituted or unsubstituted alkynyl group include an ethynyl group, a propynyl group, a butynyl group, a pentynyl group, a hexynyl group, a heptynyl group, and an octynyl group.

Examples of the substituted or unsubstituted cycloalkyl group include a cyclopropyl group, a cyclobutyl group, a cyclopentyl group, a cyclohexyl group, a methylcyclohexyl group, a cycloheptyl group, a cyclooctyl group, an adamantyl group, a cyclopentadienyl group, an indenyl group, and a fluorenyl group.

Examples of the substituted or unsubstituted aryl group include aromatic hydrocarbon groups such as a phenyl group, a methylphenyl group, a dimethylphenyl group, a diisopropylphenyl group, a dimethylisopropylphenyl group, a n-propylphenyl group, a n-butylphenyl group, a tert-butylphenyl group, a di-tert-butylphenyl group, a naphthyl group, a tetrahydronaphthyl group, a biphenyl group, a terphenyl group, a phenanthryl group, and an anthracenyl group, and hetero atom-substituted aryl groups such as a methoxyphenyl group, a dimethylaminophenyl group, a nitrophenyl group, and a trifluoromethylphenyl group.

The heteroatom-containing substituent is preferably an aryl group containing an oxygen-containing substituent, and a preferable example is, specifically, a structure in which an oxygen-containing substituent such as an alkoxy group, an aryloxy group, an alkoxyalkyl group, an aryloxyalkyl group, and a substituent in which oxygen of the above substituent is replaced with a carbonyl group or a carboxyl group is bonded to an aromatic skeleton. Among such substituents, a substituent in which an alkoxy group or an aryloxy group is bonded to an aromatic skeleton is preferable, and a substituent in which an alkoxy group is bonded to an aromatic skeleton is more preferable. The number of carbon atoms in the oxygen-containing substituent is preferably 1 to 10, more preferably 1 to 8, and even more preferably 1 to 6. More specifically, preferably examples in addition to the methoxyphenyl group include an ethoxyphenyl group, a propyloxyphenyl group, an isopropyloxyphenyl group, a butoxyphenyl group, and a phenoxyphenyl group. An aryl group containing such an oxygen-containing substituent may be used particularly preferably for $R^1$ and $R^2$.

Examples of the substituted or unsubstituted hetero atom-containing hydrocarbon group include hetero atom-containing alkyl groups such as a methoxymethyl group, a methoxyethyl group, a benzyloxy group, an ethoxymethyl group, an ethoxyethyl group, an acetyl group, a benzoyl group, and heteroaryl groups such as a furyl group, a pyrrolyl group, a thienyl group, a pyrazolyl group, a pyridyl group, a carbazolyl group, an imidazolyl group, a dimethylfuryl group, a N-methylpyrrolyl group, a N-phenylpyrrolyl group, a diphenylpyrrolyl group, a thiazolyl group, a quinolyl group, a benzofuryl group, a triazolyl group, and a tetrazolyl group.

$R^3$ to $R^8$ when being substituents other than hydrogen can be selected from the substituents exemplified for $R^3$ to $R^8$ above. $R^3$ to $R^8$ are more preferably substituents selected from hydrocarbon groups and heteroatom-containing hydrocarbon groups, and are hydrocarbon groups in particular.

<Specific examples of ester compound (A)>

While specific examples of the ester compound (A) of the present invention are shown below, the ester compound (A) of the present invention is not limited thereto.

[Formula 4]

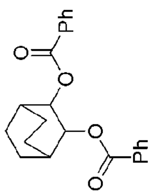
1-1

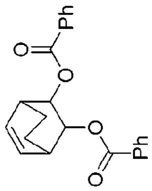
1-2

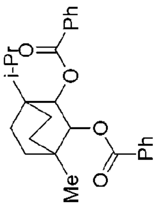
1-3

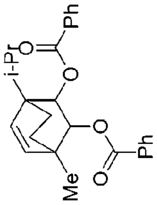
1-4

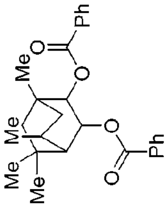
1-5

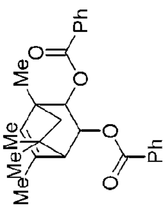
1-6

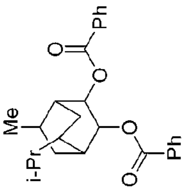
1-7

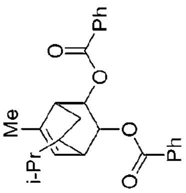
1-8

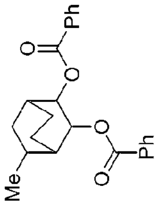
1-9

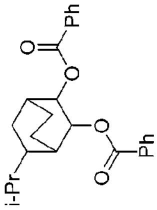
1-10

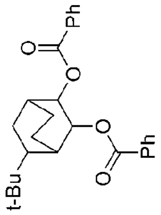
1-11

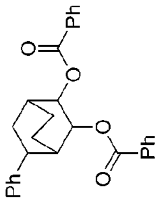
1-12

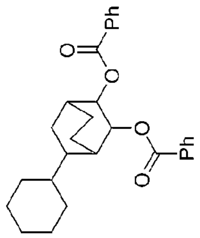
1-13

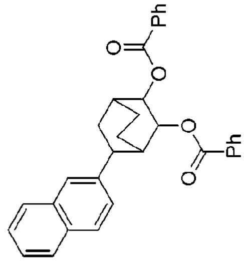
1-14

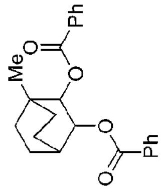
1-15

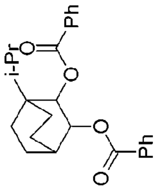
1-16

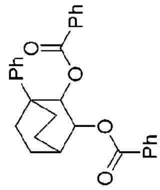
1-17

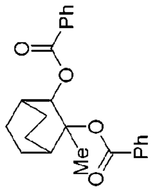
1-18

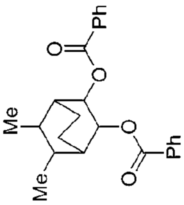
1-19

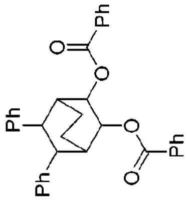
1-20

[Formula 5]

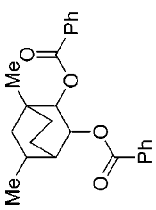
1-21

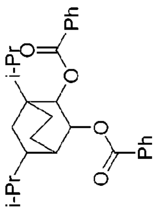
1-22

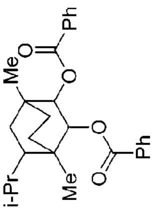
1-23

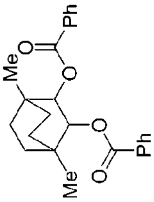
1-24

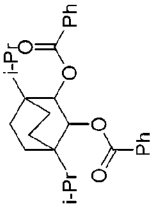
1-25

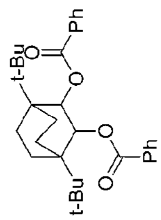
1-26

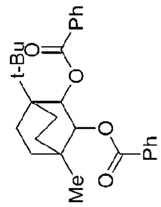
1-27

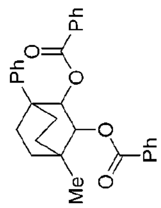
1-28

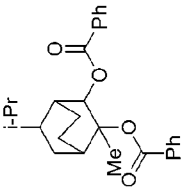
1-29

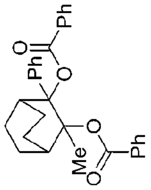
1-30

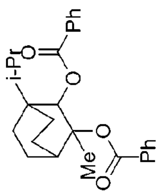
1-31

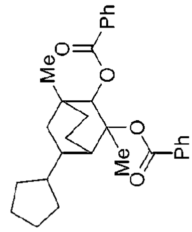
1-32

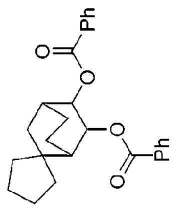
1-33

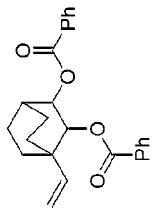
1-34

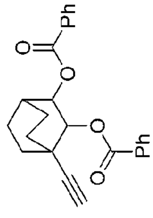
1-35

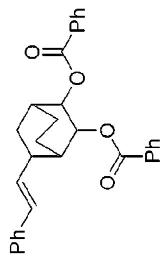
1-36

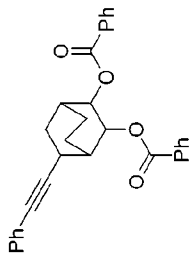
1-37

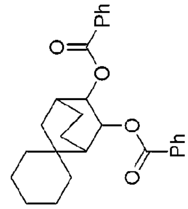
1-38

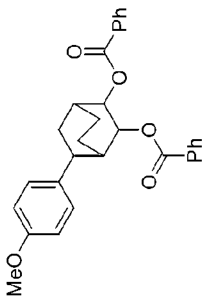
1-39

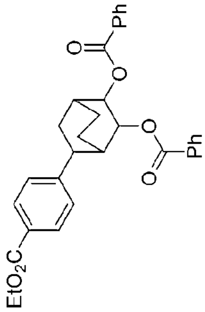
1-40

[Formula 6]

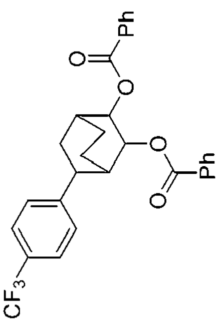

1-41

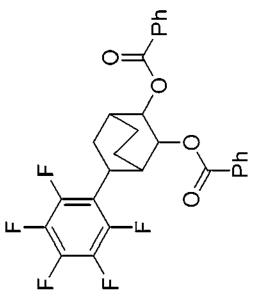

1-42

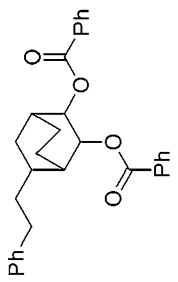

1-43

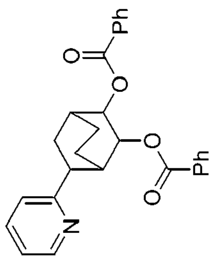

1-44

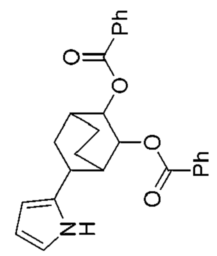

1-45

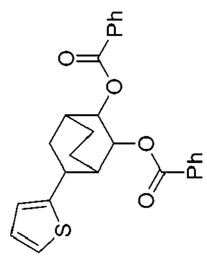

1-46

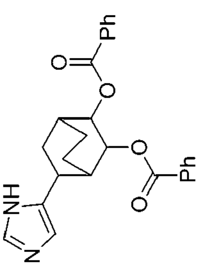

1-47

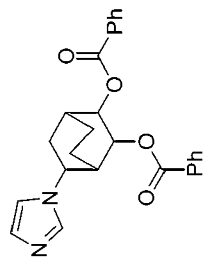

1-48

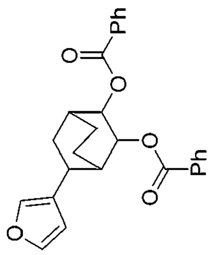

1-49

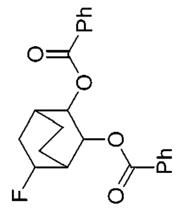

1-50

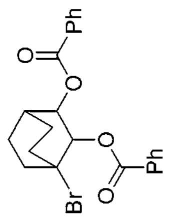

1-51

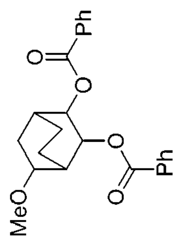

1-52

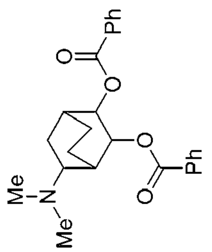

1-53

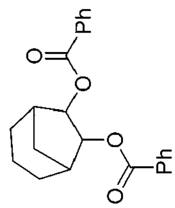

1-54

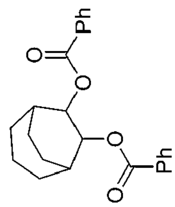

1-55

[Formula 7]

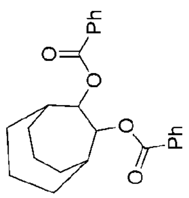
1-56

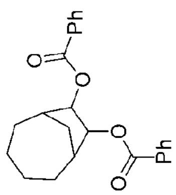
1-57

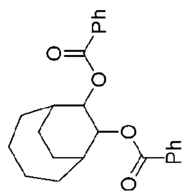
1-58

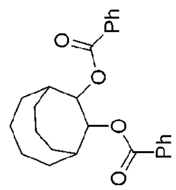
1-59

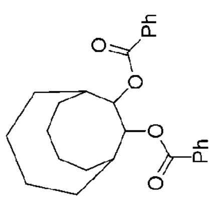
1-60

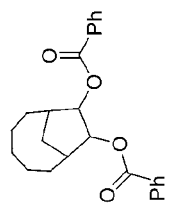
1-61

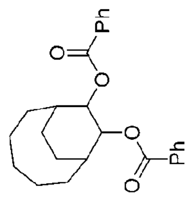
1-62

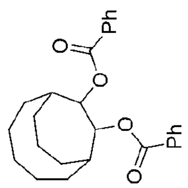
1-63

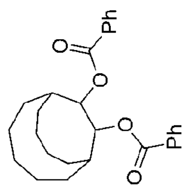
1-64

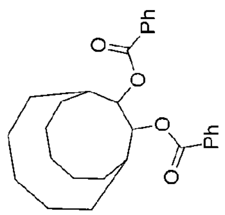
1-65

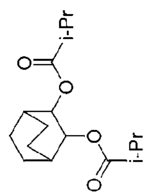
1-66

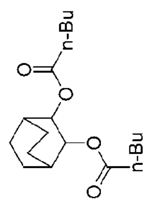
1-67

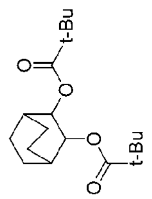
1-68

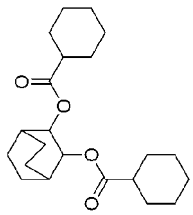
1-69

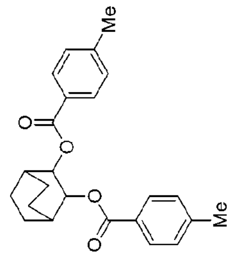
1-70

[Formula 8]

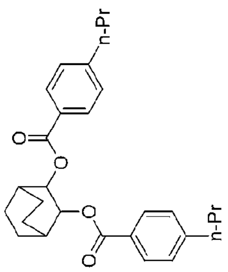

1-71

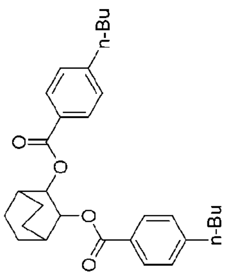

1-72

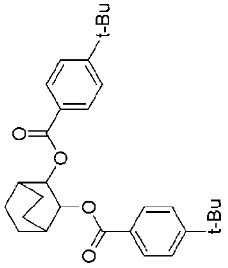

1-73

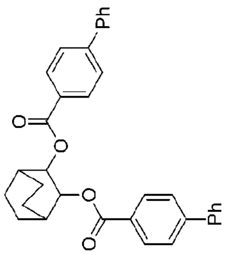

1-74

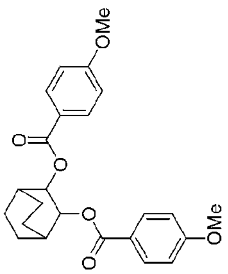

1-75

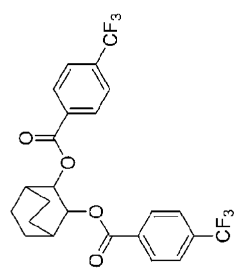

1-76

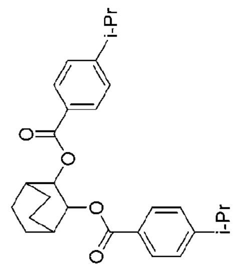

1-77

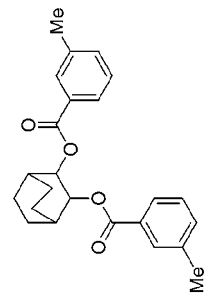

1-78

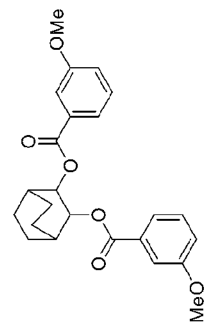

1-79

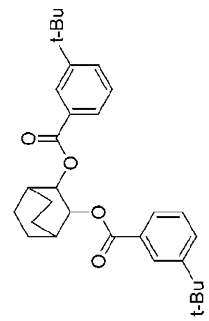

1-80

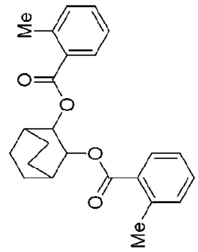

1-81

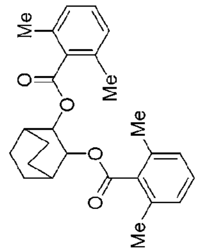

1-82

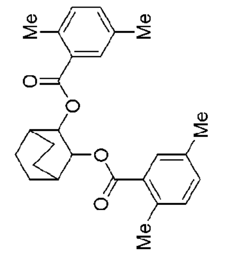

1-83

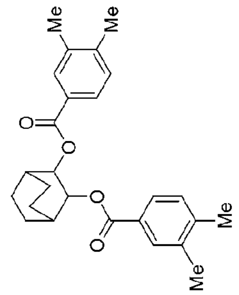

1-84

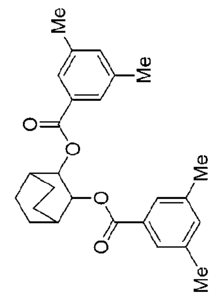

1-85

[Formula 9]

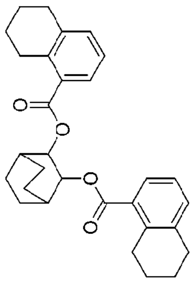
1-86

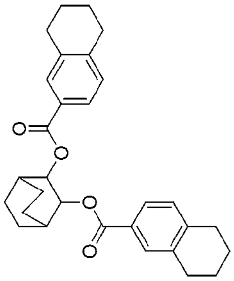
1-87

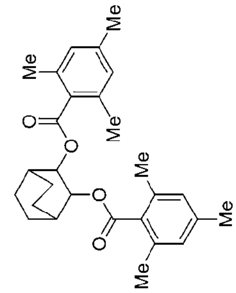
1-88

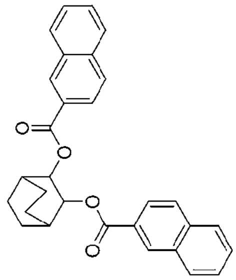
1-89

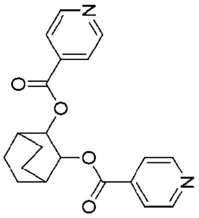
1-90

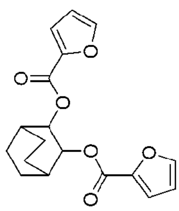
1-91

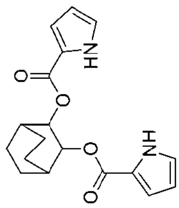
1-92

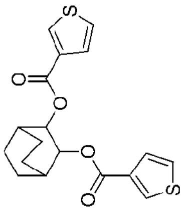
1-93

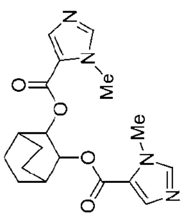
1-94

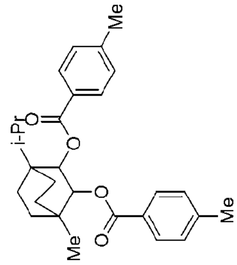
1-95

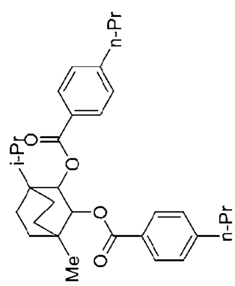
1-96

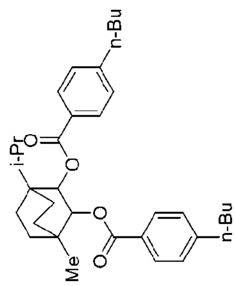
1-97

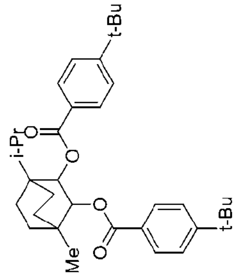
1-98

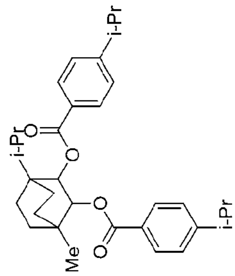
1-99

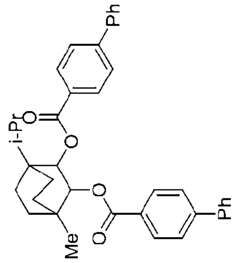
1-100

[Formula 10]

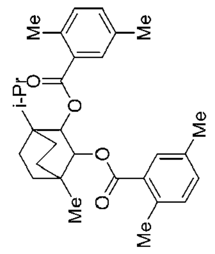
1-105

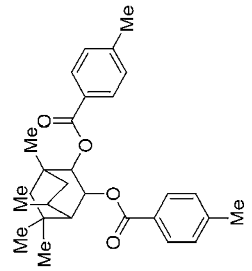
1-110

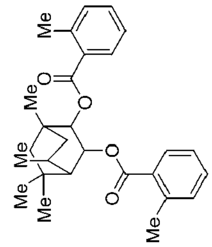
1-115

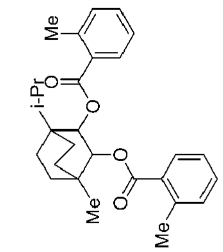
1-104

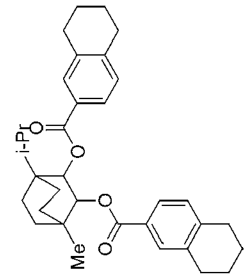
1-109

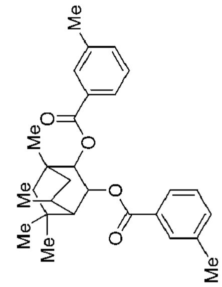
1-114

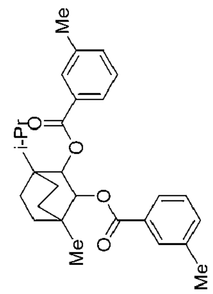
1-103

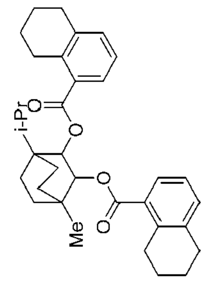
1-108

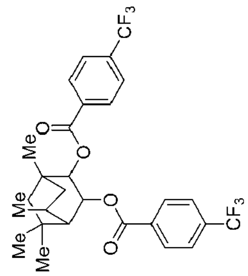
1-113

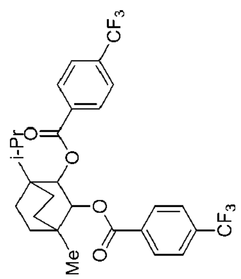
1-102

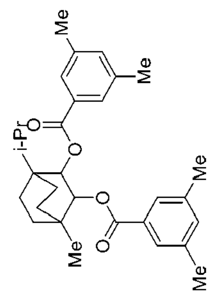
1-107

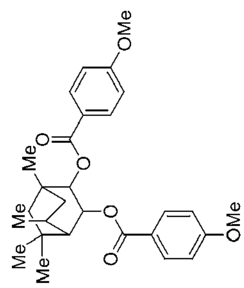
1-112

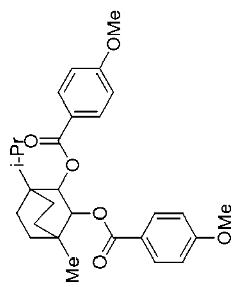
1-101

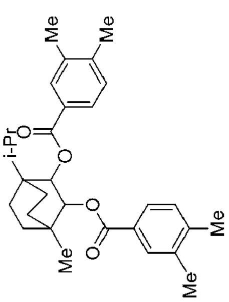
1-106

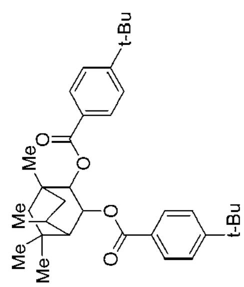
1-111

[Formula 11]

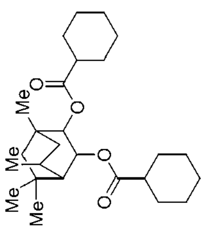

1-116

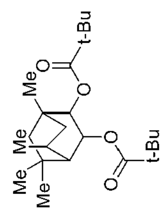

1-117

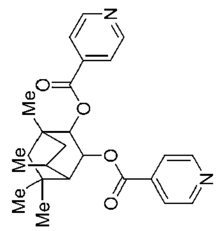

1-118

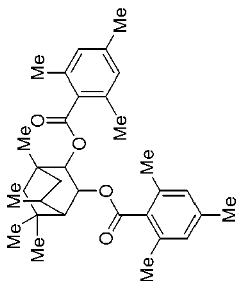

1-119

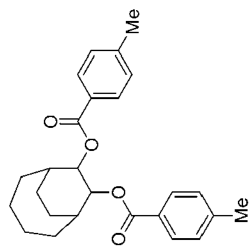

1-120

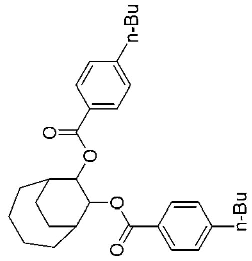

1-121

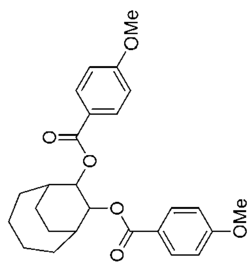

1-122

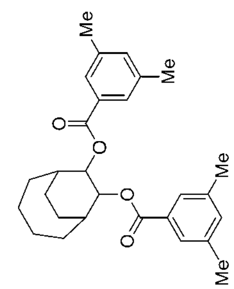

1-123

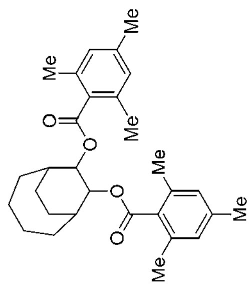

1-124

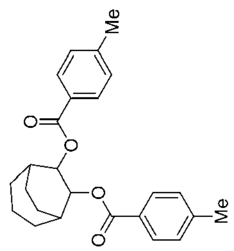

1-125

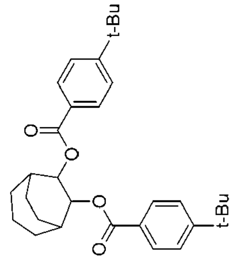

1-126

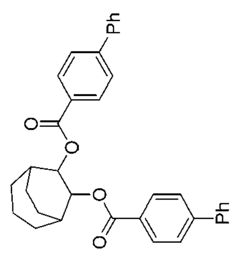

1-127

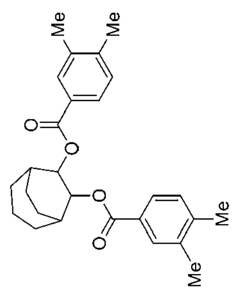

1-128

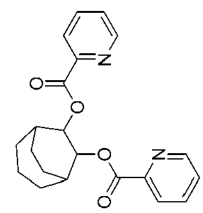

1-129

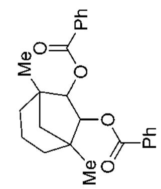

1-130

[Formula 12]

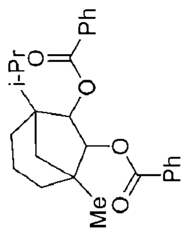
1-131

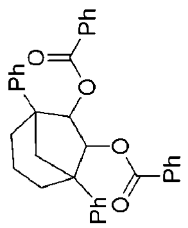
1-132

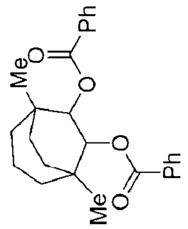
1-133

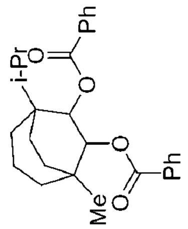
1-134

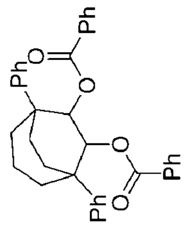
1-135

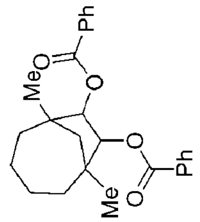
1-136

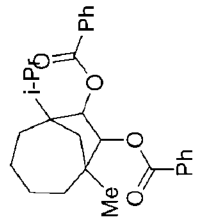
1-137

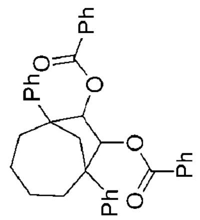
1-138

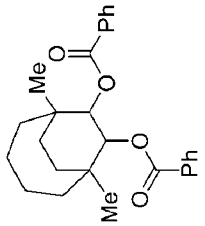
1-139

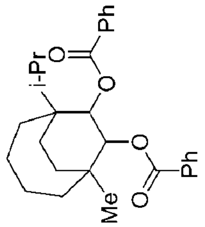
1-140

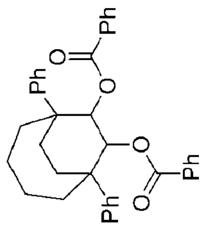
1-141

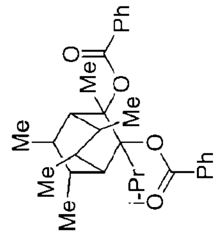
1-142

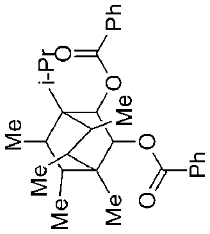
1-143

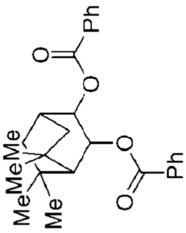
1-144

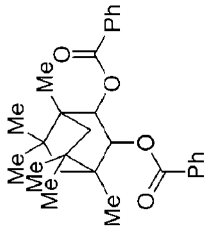
1-145

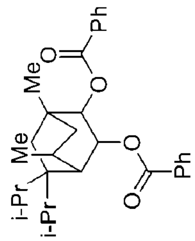
1-146

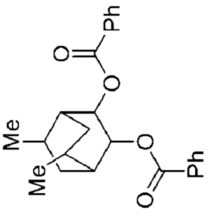
1-147

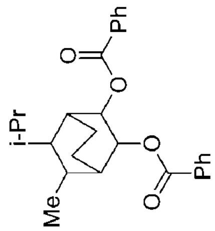
1-148

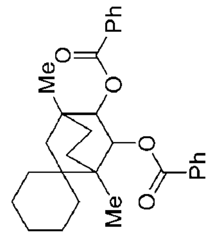
1-149

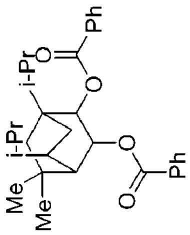
1-150

[Formula 13]

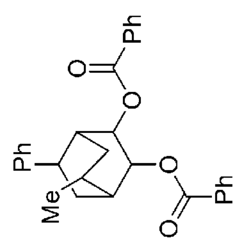

1-151

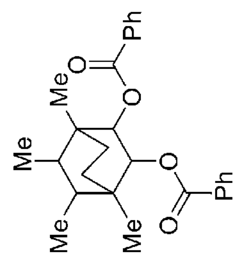

1-152

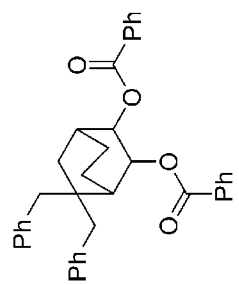

1-153

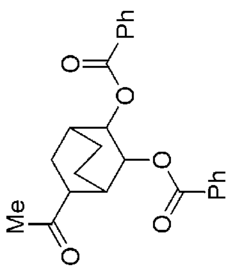

1-154

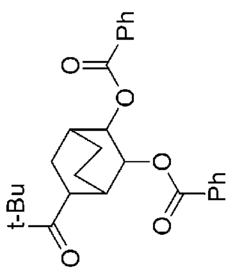

1-155

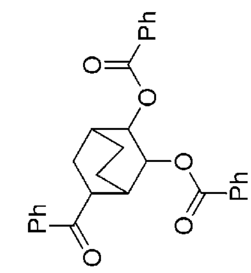

1-156

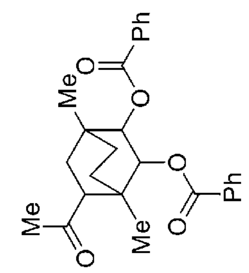

1-157

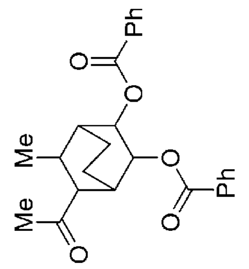

1-158

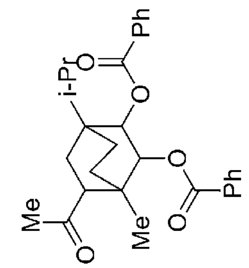

1-159

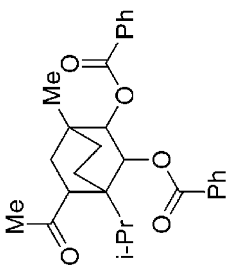

1-160

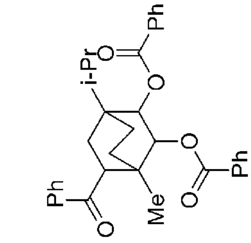

1-161

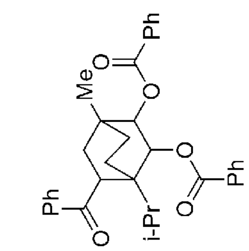

1-162

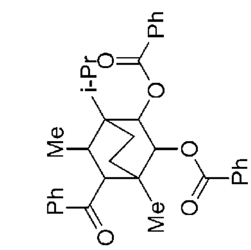

1-163

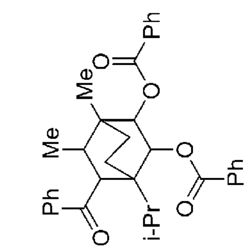

1-164

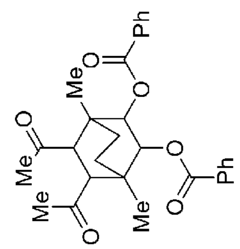

1-165

In the structural formulae above, a methyl group is denoted as "Me", an ethyl group is denoted as "Et", a propyl group is denoted as "Pr", a butyl group is denoted as "Bu", and a phenyl group is denoted as "Ph". Also, [n] represents "normal", [i] represents "iso", and "t" represents "tertially".

In the ester compound of the present invention, the $OCOR^1$ group and $OCOR^2$ group bonded to the alicyclic structure may form a cis structure or a trans structure derived from the alicyclic structure, and the ester compound of the cis structure is preferably the main component. The main component here means that the content of the compound with a cis structure exceeds 50 mol% and is preferably 70 mol% or more.

One suitable application of the ester compound (A) of the present invention is a Lewis base (an internal donor) component of the solid titanium catalyst component. Although the reason why the ester compound (A) of the present invention is suitable as the internal donor component is currently not clear, the present inventors presume the reason as follows.

The ester compound (A) used in the present invention has, as described above, a specific multicyclic structure, and it is thus presumed that the compound has suitable rigidity as a compound, and the structural displacement is relatively small. On the other hand, the structure can be understood as concomitantly having also moieties that allows rather flexible movement. Accordingly, it seems that when the ester compound (A) coordinates to a titanium compound or a magnesium compound that will be described below, it maintains a stable structure, and variations of stereospecificity and polymerization reaction activity as a catalyst during an olefin polymerization reaction are small. The flexible structural moieties are expected to alleviate strain resulting from the cyclic structure, and thus may have a buffer-like function against changes of the reaction environment. From these viewpoints, it seems that a highly stereoregular polymer can be obtained in a highly active manner. Also, from such viewpoints, it can be presumed that the ester compound (A) potentially provides even a high molecular weight component.

On the other hand, in the case of a stable structure with small structural displacement, there were initially concerns about a narrow molecular weight distribution, but as shown in the Examples below, the method of the present invention enables a polymer having a broad molecular weight distribution to be produced. This, as presumed by the present inventors, is due to the possibility that, in the case of the ester compound (A), small variations of cyclic structures and combinations of such variations of respective ring structures highly affect the molecular weight of the resulting polymer, and having multiple ring structures results in a variety of combinations of stereoisomeric structures that the respective rings can take (such as a chair conformation and a boat conformation).

<Method for producing ester compound (A)>

The method for producing the ester compound (A) of the present invention is not particularly limited. For example, the ester compound (A) can be obtained by the reaction of a dihydroxylation of a corresponding olefin, followed by an esterification of the diol obtained by the dihydroxylation. In addition to that, the ester compound (A) can be also obtained by the successive reaction of introducing a carbonate group to a particular polycyclic compound such as cyclohexadiene, a hydrolysis of the carbonate group, and then an esterification of the diol obtained by the hydrolysis. More specifically, the ester compound (A) can be produced as follows.

<<Synthesis of olefin>>

The olefin shown in the following formula (21) can be synthesized by, for example, a Diels-Alder reaction of cyclohexadiene and methyl vinyl ketone (Non-Patent Literature 1). For the diene, a dimer of a diene (e.g., dicyclopentadiene), which is a precursor, can be also used as a raw material.

<<Synthesis of diol>>

A diol form, which is an ester precursor, can be produced using the corresponding olefin as a raw material. For example, a reaction of an olefin with potassium permanganate (Non-Patent Literature 4) or osmium tetroxide (Non-Patent Literature 5) enables a diol form to be directly obtained.

As a different method, a diol form can be obtained by epoxidizing an olefin moiety using m-chloroperoxybenzoic acid (Non-Patent Literature 6); tert-butyl peroxide (Non-Patent Literature 7); dimethyldioxirane (Non-Patent Literature 8); formic acid and hydrogen peroxide solution (Non-Patent Literature 9); hydrogen peroxide solution and a molybdenum catalyst; or hydrogen peroxide solution and a tungsten catalyst (Non-Patent Literature 10) followed by an acidic or alkali hydrolysis reaction.

A diol compound can also be obtained after a hydrolysis of the cyclic carbonate that is obtained from the diene. The details are as follows.

The cyclic carbonate, which is a diol precursor, can be produced by a Diels-Alder reaction of the corresponding diene and vinylene carbonate (Non-Patent Literature 19). As described above, for the diene, a dimer of the diene, which is a precursor, also can be used as the raw material.

Acidic- or alkali-hydrolyzing the cyclic carbonate of the formula (24) enables the diol (the formula (23)) to be obtained (Non-Patent Literature 19).

<<Synthesis of ester>>

An ester corresponding to the formula (1) can be synthesized by allowing the diol (the formula (23)) and an

acid chloride to react in the presence of a base (the formula 25). Examples of the base that can be used include, but are not particularly limited to, sodium hydroxide, potassium hydroxide, and amine bases. The ester can be synthesized also by a synthesis method in which a diol and a carboxylic acid are allowed to react in the presence of an acid catalyst or by using a condensing reagent such as DCC (Non-Patent Literature 11) (the formula (26)). When one equivalent of an acid chloride or carboxylic acid is allowed to react with a diol form (the formula (3')), an isomer corresponding to the formula (24) may be formed, but subsequently allowing an acid chloride or carboxylic acid to react enables a compound corresponding to the formula (1) to be obtained. At this time, $R^1$ and $R^2$ may be the same or different. The ester can be synthesized also by allowing a diol form to react with a carboxylic acid in the presence of an azocarboxylic ester and triphenylphosphine (Non-Patent Literature 12).

The ester compound (A) of the present invention is suitable as the Lewis base component of the solid titanium catalyst component, as mentioned previously, but is not limited to this application. Needless to say, the ester compound can be possibly applied to known additive applications such as additives to various resins, cosmetics and external preparations for the skin, microbicidal compositions, antioxidants, and chelators.

## Examples

Methods for synthesizing the ester compound of the present invention will be exemplified in the following Examples. The compounds disclosed in the following Examples represent some stereoisomers, and may include other stereoisomers.

In the structural formulae, "Me" represents a methyl group, "n-Pr" represents a normal propyl group, "iPr" represents an isopropyl group, "n-Bu" represents a normal butyl group, "tBu" represents a tertiary butyl group, and "Ph" represents a phenyl group.

In the case of $^1$H-NMR measurement, a JNM-EX270 nuclear magnetic resonance apparatus manufactured by JEOL Ltd. was used, and a small amount of tetramethyl silane was added to a deuterated chloroform solvent.

The conditions included a measurement temperature of room temperature, an observed nucleus of $^1$H (270 MHz), a sequence of a single pulse, a 45° pulse, a repeating time of 5.5 seconds or more, and an integrated frequency of 16 to 64 times or more. As a reference chemical shift, the hydrogen of the tetramethylsilane was set to 0 ppm. Peaks of, for example, $^1$H derived from an organic acid compound were assigned by a conventional method.

[Example 1] (Synthesis of ester compound)

<Synthesis of compound 1>

A compound 1 shown below was synthesized by the method described below.

[Formula 14]

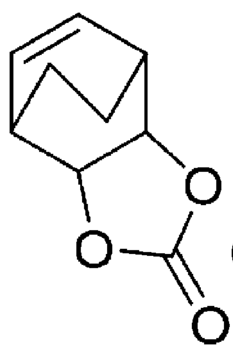

(Compound 1)

In a nitrogen atmosphere, 8.0 g of 1,3-cyclohexadiene and 8.6 g of vinylene carbonate were added to a 30 ml pressure-resistant vessel, and stirred while being heated such that the internal temperature was 220°C, and stirring was continued for 6 hours. After the mixture was cooled to room temperature, 5 ml of hexane was added, the mixture was stirred, and then solids were filtered off. The resulting solids were washed with hexane and then dried to give 8.5 g of a compound 1.

<Synthesis of compound 2>

A compound 2 shown below was synthesized by the method described below.

[Formula 15]

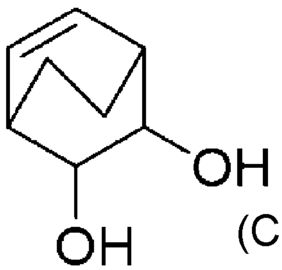

(Compound 2)

First, 16.6 g of the compound 1, 40.0 g of sodium hydroxide, and 100 ml of pure water were added to a 50 ml three-neck flask and stirred while being heated such that the internal temperature was 100°C, and stirring was continued for 6 hours. After the mixture was cooled to room temperature, 12 mol/L of concentrated hydrochloric acid was added at 50°C or lower for neutralization. The reaction solution was extracted three times with ethyl acetate, and the organic layer was dried over sodium sulfate and concentrated to give 14.0 g of a compound 2.

<Synthesis of compound 3>

A compound 3 shown below was synthesized by the method described below.

[Formula 16]

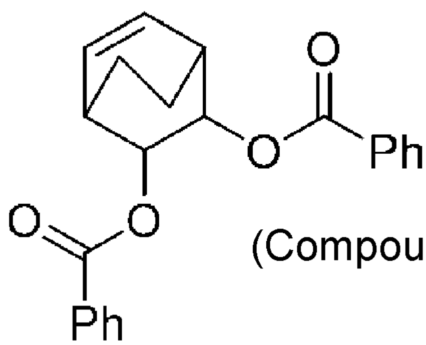

(Compound 3)

In a nitrogen atmosphere, 14.0 g of the compound 2, 42.2 g of benzoyl chloride, and 200 ml of pyridine were added to a 200 ml three-neck flask and stirred while being heated such that the internal temperature was 60°C, and stirring was continued for 6 hours. After pyridine was distilled off, chloroform was added, the mixture was washed with 2 mol/L of hydrochloric acid and 2 mol/L of an aqueous sodium hydroxide solution, and then the organic layer was dried over magnesium sulfate. After being concentrated, the organic layer was recrystallized from hexane to give 13.1 g of a compound 3.

$^1H$ NMR (270 MHz, $CDCl_3$, TMS as an internal standard): 7.83-7.80 (m, 4H), 7.44-7.41 (m, 2H), 7.28-7.20 (m, 4H), 6.41-6.39 (m, 2H), 5.35 (s, 2H), 3.00 (s, 2H), 1.71-1.40 (m,4H).

The melting point of the resulting compound 3 was measured with a differential scanning calorimeter (DSC-60A manufactured by SHIMADZU CORPORATION, start temperature: 25°C, end temperature: 300°C, heating rate: 10°C/min) and was 95°C.

[Example 2] (Synthesis of ester compound)

A compound 4 shown below was synthesized by the method described below.

[Formula 17]

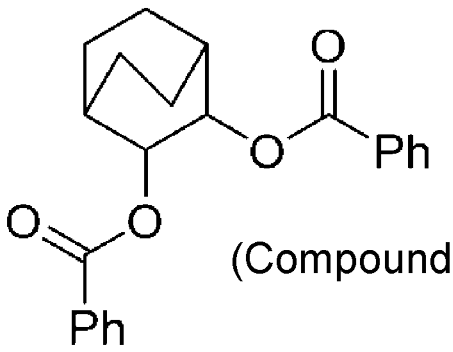

(Compound 4)

In a nitrogen atmosphere, 7.0 g of the compound 3 obtained in Example 1, 3.6 g of Pd/C (manufactured by Evonik, 5% Pd, water content: 58.6%), and 100 ml of ethyl acetate were added to a 300 ml three-necked flask, and stirred at room temperature. Hydrogen at 1 atm was introduced into the reaction vessel to change the nitrogen atmosphere to a hydrogen atmosphere, and stirring was continued for 15 hours. After the mixture was filtered through celite, the filtrate was concentrated. The filtrate was recrystallized from hexane to give 6.6 g of a compound 4.

$^1$H NMR (270 MHz, $CDCl_3$, TMS as an internal standard): 7.91-7.88 (m, 4H), 7.49-7.42 (m, 2H), 7.30-7.24 (m, 4H), 5.34 (s, 2H), 3.00 (s, 2H), 2.09-2.05 (m, 4H), 1.71 (brs, 4H), 1.54-1.52 (m, 2H).

The melting point of the resulting compound 4 was measured with a differential scanning calorimeter (DSC-60A manufactured by SHIMADZU CORPORATION, start temperature: 25°C, end temperature: 300°C, heating rate: 10°C/min) and was 105°C.

[Example 3] (Synthesis of ester compound)

<Synthesis of compound 5>

A compound 5 shown below was synthesized by the method described below.

[Formula 18]

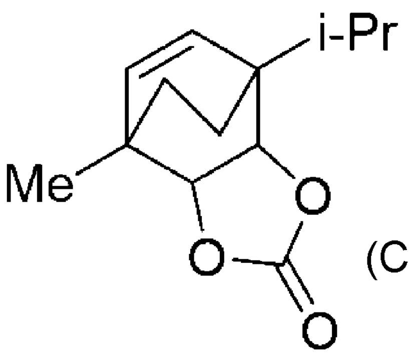

(Compound 5)

In a nitrogen atmosphere, 13.6 g of α-terpinene and 8.6 g of vinylene carbonate were added to a 30 ml pressure-resistant vessel, and stirred while being heated such that the internal temperature was 220°C, and stirring was continued for 6 hours. After the mixture was cooled to room temperature, 5 ml of hexane was added, the mixture was stirred, and then solids were filtered off. The resulting solids were washed with hexane and then dried to give 14.7 g of a compound 5.

<Synthesis of compound 6>

A compound 6 shown below was synthesized by the method described below.

[Formula 19]

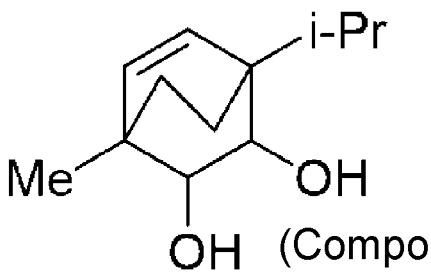

(Compound 6)

First, 4.5 g of the compound 5, 8.0 g of sodium hydroxide, and 30 ml of pure water were added to a 50 ml three-neck flask and stirred while being heated such that the internal temperature was 100°C, and stirring was continued for 6 hours. After the mixture was cooled to room temperature, 12 mol/L of concentrated hydrochloric acid was added at 50°C or lower for neutralization. The reaction solution was extracted three times with ethyl acetate, and the organic layer was dried over sodium sulfate and concentrated, and solids were recovered to give 3.9 g of a compound 6.

<Synthesis of compound 7>

A compound 7 shown below was synthesized by the method described below.

[Formula 20]

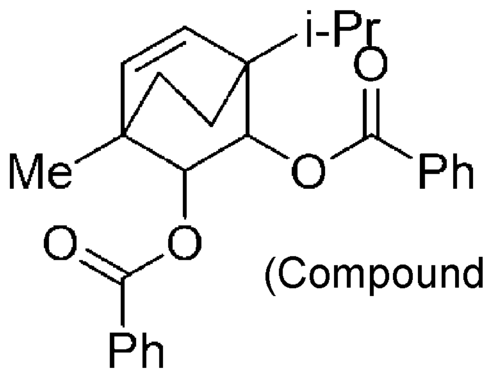

(Compound 7)

In a nitrogen atmosphere, 3.9 g of the compound 6, 8.4 g of benzoyl chloride, and 100 ml of pyridine were added to a 200 ml three-neck flask and stirred while being heated such that the internal temperature was 60°C, and stirring was continued for 6 hours. After pyridine was distilled off, chloroform was added, the mixture was washed with 2 mol/L of hydrochloric acid and 2 mol/L of an aqueous sodium hydroxide solution, and then the organic layer was dried over magnesium sulfate. After the organic layer was concentrated, solids were filtered off, washed with hexane, and then dried to give 3.0 g of a compound 7.

$^1H$ NMR (270 MHz, $CDCl_3$, TMS as an internal standard): 7.79-7.68 (m, 4H), 7.43-7.29 (m, 2H), 7.25-7.07 (m, 4H), 6.28-6.09 (m, 2H), 5.42-5.34 (m, 2H), 2.23-2.12 (m, 1H), 1.63-1.25 (m, 4H), 1.17 (s, 3H), 1.02 (d, J = 7.0 Hz, 3H), 0.92 (d, J = 7.0 Hz, 3H).

The melting point of the resulting compound 7 was measured with a differential scanning calorimeter (DSC-60A manufactured by SHIMADZU CORPORATION, start temperature: 25°C, end temperature: 300°C, heating rate: 10°C/min) and was 137°C.

[Example 4] (Synthesis of ester compound)

<Synthesis of compound 8>

A compound 8 shown below was synthesized by the method described below.

[Formula 21]

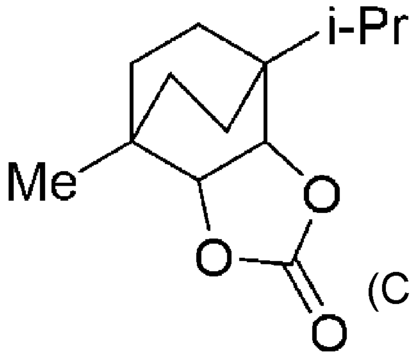

(Compound 8)

In a nitrogen atmosphere, 4.5 g of the compound 5, 4.3 g of Pd/C (manufactured by Evonik, 5% Pd, water content: 58.6%), and 100 ml of ethyl acetate were added to a 200 ml three-necked flask, and stirred at room temperature. Hydrogen at 1 atm was introduced into the reaction vessel to change the nitrogen atmosphere to a hydrogen atmosphere, and stirring was continued for 15 hours. After the mixture was filtered through celite, the filtrate was concentrated, and solids were recovered to give 4.5 g of a compound 8.

<Synthesis of compound 9>

A compound 9 shown below was synthesized by the method described below.

[Formula 22]

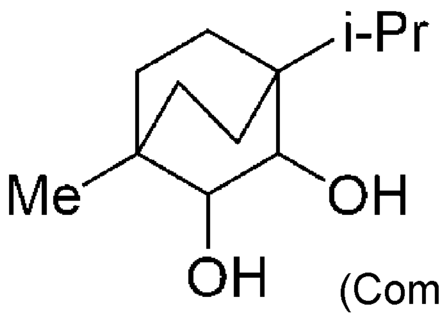

(Compound 9)

First, 4.5 g of the compound 8, 8.0 g of sodium hydroxide, and 30 ml of pure water were added to a 50 ml three-neck flask and stirred while being heated such that the internal temperature was 100°C, and stirring was continued for 6 hours. After the mixture was cooled to room temperature, 12 mol/L of concentrated hydrochloric acid was added at 50°C or lower for neutralization. The reaction solution was extracted three times with ethyl acetate, and the organic layer was dried over sodium sulfate and concentrated to give 3.8 g of a compound 9.

<Synthesis of compound 10>

A compound 10 shown below was synthesized by the method described below.

[Formula 23]

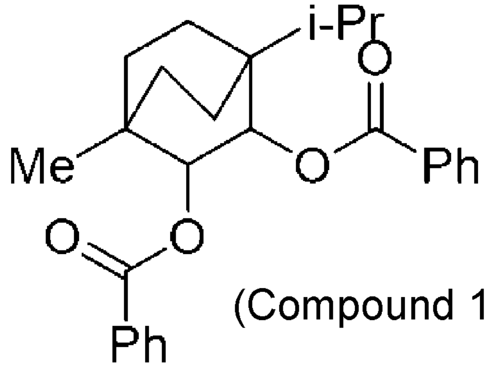

(Compound 10)

In a nitrogen atmosphere, 2.0 g of the compound 9, 3.2 g of benzoyl chloride, and 50 ml of pyridine were added to a 200 ml three-neck flask and stirred while being heated such that the internal temperature was 60°C, and stirring was continued for 6 hours. After pyridine was distilled off, chloroform was added, the mixture was washed with 2 mol/L of hydrochloric acid and 2 mol/L of an aqueous sodium hydroxide solution, and then the organic layer was dried over magnesium sulfate. After being concentrated, the organic layer was purified by silica gel column chromatography and dried to give 3.6 g of a compound 10.

$^1H$ NMR (270 MHz, $CDCl_3$, TMS as an internal standard): 7.87-7.74 (m, 4H), 7.45-7.12 (m, 6H), 5.49-5.46 (m, 1H), 5.26-5.25 (m, 1H), 2.10-0.82 (m, 18H).

The melting point of the resulting compound 10 was measured with a differential scanning calorimeter (DSC-60A manufactured by SHIMADZU CORPORATION, start temperature: 25°C, end temperature: 300°C, heating rate: 10°C/min) and was 107°C.

[Example 5] (Synthesis of ester compound)

<Synthesis of compound 11>

A compound 11 shown below was synthesized by the method described below.

[Formula 24]

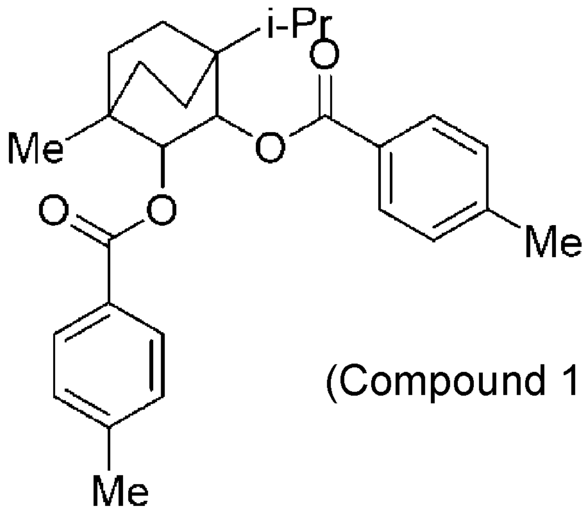

(Compound 11)

In a nitrogen atmosphere, 4.0 g of the compound 9, 9.3 g of 4-methylbenzoyl chloride, and 50 ml of pyridine were added to a 200 ml three-neck flask and stirred while being heated such that the internal temperature was 60°C,

and stirring was continued for 6 hours. After pyridine was distilled off, chloroform was added, the mixture was washed with 2 mol/L of hydrochloric acid and 2 mol/L of an aqueous sodium hydroxide solution, and then the organic layer was dried over magnesium sulfate. After being concentrated, the organic layer was purified by silica gel column chromatography and dried to give 2.9 g of a compound 11.

$^1$H NMR (270 MHz, $CDCl_3$, TMS as an internal standard): 7.76 (d, J = 7.8 Hz, 2H), 7.66 (d, J= 7.8 Hz, 2H), 7.09 (d, J = 8.6 Hz, 2H), 6.96 (d, J = 8.4 Hz, 2H), 5.47-5.23 (m, 2H), 2.36 (s, 3H), 2.30 (s, 3H), 2.09-1.26 (m, 11H), 0.95-0.81 (m, 6H).

[Example 6] (Synthesis of ester compound)

<Synthesis of compound 12>

A compound 12 shown below was synthesized by the method described below.

[Formula 25]

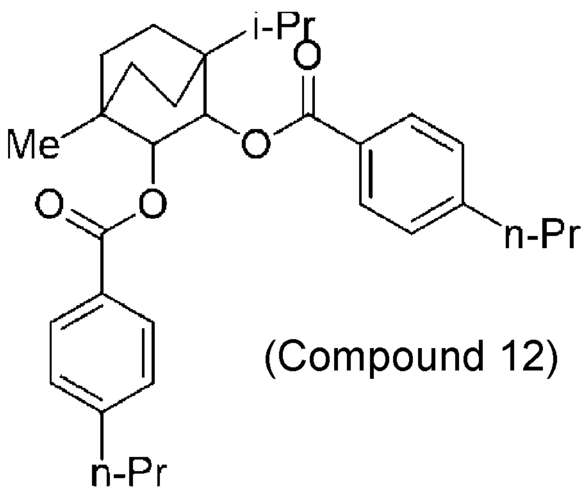

(Compound 12)

In a nitrogen atmosphere, 4.0 g of the compound 9, 11.0 g of 4-n-propylbenzoyl chloride, and 50 ml of pyridine were added to a 200 ml three-neck flask and stirred while being heated such that the internal temperature was 60°C, and stirring was continued for 6 hours. After pyridine was distilled off, chloroform was added, the mixture was washed with 2 mol/L of hydrochloric acid and 2 mol/L of an aqueous sodium hydroxide solution, and then the organic layer was dried over magnesium sulfate. After being concentrated, the organic layer was purified by silica gel column chromatography and dried to give 2.3 g of a compound 12.

$^1$H NMR (270 MHz, $CDCl_3$, TMS as an internal standard): 7.76 (d, J = 8.4 Hz, 2H), 7.67 (d, J= 8.4 Hz, 2H), 7.07 (d, J = 7.8 Hz, 2H), 6.93 (d, J = 8.1 Hz, 2H), 5.47-5.23 (m, 2H), 2.51-2.48 (m, 4H), 2.10-1.26 (m, 16H), 0.94-0.80 (m, 12H).

The melting point of the resulting compound 12 was measured with a differential scanning calorimeter (DSC-60A manufactured by SHIMADZU CORPORATION, start temperature: 25°C, end temperature: 300°C, heating rate: 10°C/min) and was 70°C.

[Example 7] (Synthesis of ester compound)

<Synthesis of compound 13>

A compound 13 shown below was synthesized by the method described below.

[Formula 26]

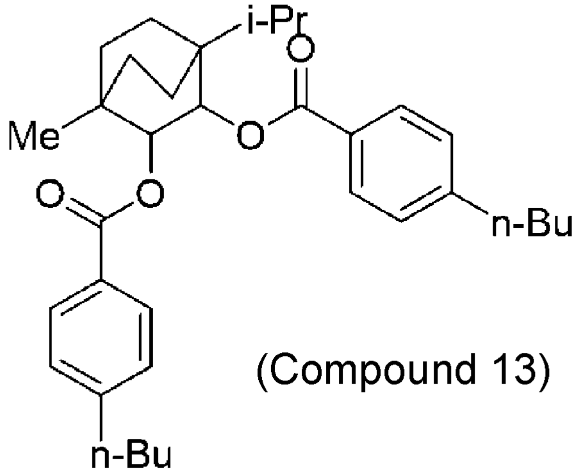

(Compound 13)

In a nitrogen atmosphere, 4.0 g of the compound 9, 11.8 g of 4-n-butylbenzoyl chloride, and 50 ml of pyridine were added to a 200 ml three-neck flask and stirred while being heated such that the internal temperature was 60°C, and stirring was continued for 6 hours. After pyridine was distilled off, chloroform was added, the mixture was washed with 2 mol/L of hydrochloric acid and 2 mol/L of an aqueous sodium hydroxide solution, and then the organic layer was dried over magnesium sulfate. After being concentrated, the organic layer was purified by silica gel column chromatography and dried to give 7.9 g of a compound 13.

$^1H$ NMR (270 MHz, $CDCl_3$, TMS as an internal standard): 7.76 (d, J = 7.8 Hz, 2H), 7.66 (d, J= 8.1 Hz, 2H), 7.08 (d, J = 7.8 Hz, 2H), 6.93 (d, J = 8.4 Hz, 2H), 5.47-5.23 (m, 2H), 2.64-2.51 (m, 4H), 2.10-1.23 (m, 20H), 0.96-0.81 (m, 12H).

[Example 8] (Synthesis of ester compound)

<Synthesis of compound 14>

A compound 14 shown below was synthesized by the method described below.

[Formula 27]

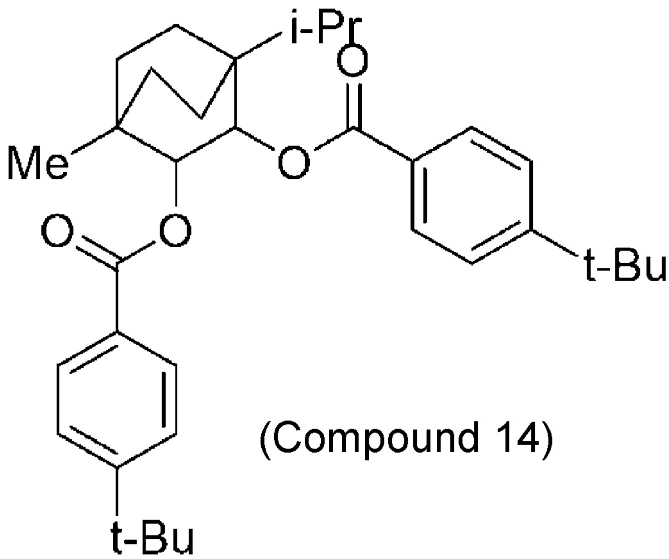

(Compound 14)

In a nitrogen atmosphere, 4.0 g of the compound 9, 11.8 g of 4-t-butylbenzoyl chloride, and 50 ml of pyridine were added to a 200 ml three-neck flask and stirred while being heated such that the internal temperature was 60°C,

and stirring was continued for 6 hours. After pyridine was distilled off, chloroform was added, the mixture was washed with 2 mol/L of hydrochloric acid and 2 mol/L of an aqueous sodium hydroxide solution, and then the organic layer was dried over magnesium sulfate. After being concentrated, the organic layer was purified by silica gel column chromatography and dried to give 1.7 g of a compound 14.

$^1$H NMR (270 MHz, $CDCl_3$, TMS as an internal standard): 7.79 (d, J = 8.6 Hz, 2H), 7.68 (d, J= 8.6 Hz, 2H), 7.31 (d, J = 8.1 Hz, 2H), 7.14 (d, J = 8.4 Hz, 2H), 5.47-5.23 (m, 2H), 2.09-1.24 (m, 30H), 0.85-0.81 (m, 6H).

The melting point of the resulting compound 14 was measured with a differential scanning calorimeter (DSC-60A manufactured by SHIMADZU CORPORATION, start temperature: 25°C, end temperature: 300°C, heating rate: 10°C/min) and was 137°C.

[Example 9] (Synthesis of ester compound)

<Synthesis of compound 15>

A compound 15 shown below was synthesized by the method described below.

[Formula 28]

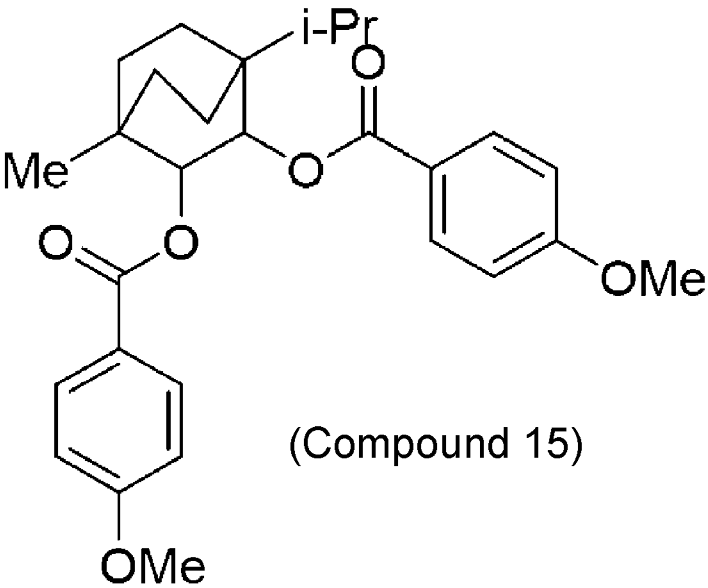

(Compound 15)

In a nitrogen atmosphere, 4.0 g of the compound 9, 10.2 g of 4-methoxybenzoyl chloride, and 50 ml of pyridine were added to a 200 ml three-neck flask and stirred while being heated such that the internal temperature was 60°C, and stirring was continued for 6 hours. After pyridine was distilled off, chloroform was added, the mixture was washed with 2 mol/L of hydrochloric acid and 2 mol/L of an aqueous sodium hydroxide solution, and then the organic layer was dried over magnesium sulfate. After being concentrated, the organic layer was purified by silica gel column chromatography and dried to give 4.7 g of a compound 15.

$^1$H NMR (270 MHz, $CDCl_3$, TMS as an internal standard): 782 (d, J = 8.9 Hz, 2H), 7.73 (d, J = 8.6 Hz, 2H), 6.78 (d, J = 8.9 Hz, 2H), 6.65 (d, J = 8.9 Hz, 2H), 5.44-5.20 (m,2H), 3.82 (s, 3H), 3.77 (s, 3H), 2.54-0.79 (m, 18H).

The melting point of the resulting compound 15 was measured with a differential scanning calorimeter (DSC-60A manufactured by SHIMADZU CORPORATION, start temperature: 25°C, end temperature: 300°C, heating rate: 10°C/min) and was 60°C.

[Example 10] (Synthesis of ester compound)

<Synthesis of compound 16>

A compound 16 shown below was synthesized by the method described below.

[Formula 29]

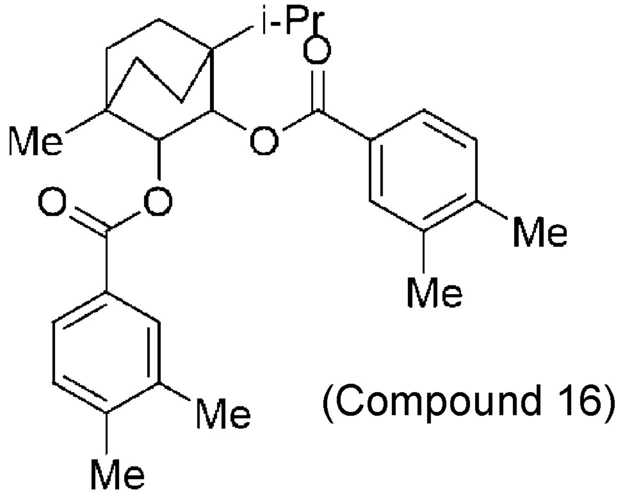

(Compound 16)

In a nitrogen atmosphere, 4.0 g of the compound 9, 10.1 g of 3,4-dimethylbenzoyl chloride, and 50 ml of pyridine were added to a 200 ml three-neck flask and stirred while being heated such that the internal temperature was 60°C, and stirring was continued for 6 hours. After pyridine was distilled off, chloroform was added, the mixture was washed with 2 mol/L of hydrochloric acid and 2 mol/L of an aqueous sodium hydroxide solution, and then the organic layer was dried over magnesium sulfate. After being concentrated, the organic layer was purified by silica gel column chromatography and dried to give 1.2 g of a compound 16.

$^1H$ NMR (270 MHz, $CDCl_3$, TMS as an internal standard): 7.65-7.45 (m, 4H), 7.09-6.94 (m, 2H), 5.46-5.23 (m, 2H), 2.67-1.30 (m, 24H), 0.84-0.75 (m, 6H).

The melting point of the resulting compound 16 was measured with a differential scanning calorimeter (DSC-60A manufactured by SHIMADZU CORPORATION, start temperature: 25°C, end temperature: 300°C, heating rate: 10°C/min) and was 92°C.

[Example 11] (Synthesis of ester compound)

<Synthesis of compound 17>

A compound 17 shown below was synthesized by the method described below.

[Formula 30]

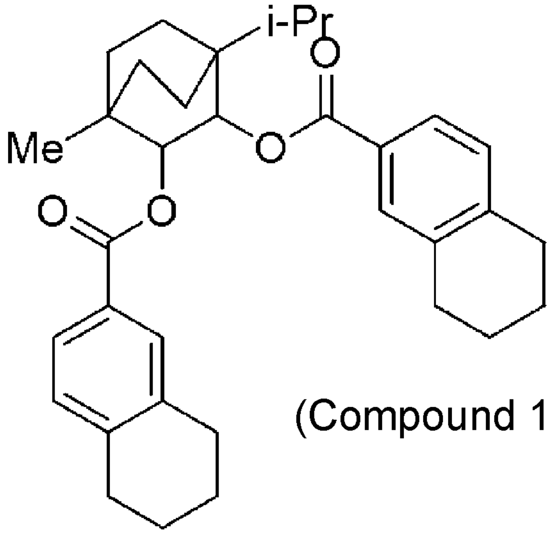

(Compound 17)

In a nitrogen atmosphere, 4.0 g of the compound 9, 11.7 g of 5,6,7,8-tetrahydronaphthalene-2-carbonyl chloride, and 50 ml of pyridine were added to a 200 ml three-neck flask and stirred while being heated such that the internal temperature was 60°C, and stirring was continued for 6 hours. After pyridine was distilled off, chloroform was added, the mixture was washed with 2 mol/L of hydrochloric acid and 2 mol/L of an aqueous sodium hydroxide solution, and then the organic layer was dried over magnesium sulfate. After being concentrated, the organic layer was purified by silica gel column chromatography and dried to give 3.8 g of a compound 17.

$^1H$ NMR (270 MHz, $CDCl_3$, TMS as an internal standard): 7.63-7.37 (m, 4H), 7.02-6.88 (m, 2H), 5.45-5.22 (m, 2H), 2.76-1.27 (m, 28H), 0.84-0.80 (m, 6H).

The melting point of the resulting compound 17 was measured with a differential scanning calorimeter (DSC-60A manufactured by SHIMADZU CORPORATION, start temperature: 25°C, end temperature: 300°C, heating rate: 10°C/min) and was 133°C.

[Example 12] (Synthesis of ester compound)

<Synthesis of compound 18 and compound 19>

Compounds 18 and 19 shown below were synthesized by the method described below.

[Formula 31]

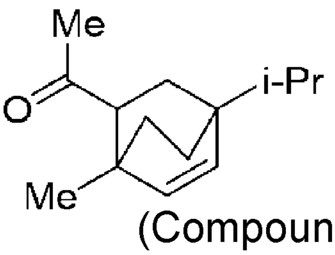

(Compound 18)

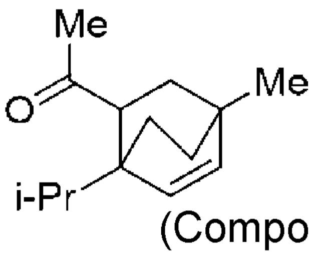

(Compound 19)

In a nitrogen atmosphere, 3.3 g of anhydrous aluminum chloride and 25 ml of dehydrated toluene were added to a 50 ml two-neck flask and stirred at room temperature. An aluminum chloride solution was prepared by slowly adding 4 ml of dehydrated THF dropwise to dissolve aluminum chloride. In a nitrogen atmosphere, 8.6 g of methyl vinyl ketone, 20.1 g of α-terpinene, and 60 ml of dehydrated toluene were added to a 300 ml three-neck flask and stirred while being

cooled in an ice bath. The previously prepared aluminum chloride solution was slowly added dropwise to the 300 ml flask and then heated to room temperature, and the mixture was stirred overnight. The mixture was cooled in an ice bath again, and 100 ml of water was added to terminate the reaction. After the mixture was separated into an organic layer and an aqueous layer, the organic layer was washed with 50 ml of water, a saturated aqueous sodium bicarbonate solution, and brine in this order, and the organic layer was dried over magnesium sulfate and then concentrated in a rotary evaporator. The resulting crude product was purified by silica gel column chromatography to give 18.4 g of an isomeric mixture of a compound 18 and a compound 19.

<Synthesis of compound 20 and compound 21>

Compounds 20 and 21 shown below were synthesized by the method described below.

[Formula 32]

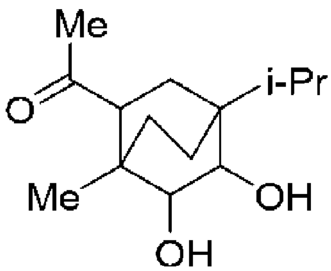

(Compound 20)

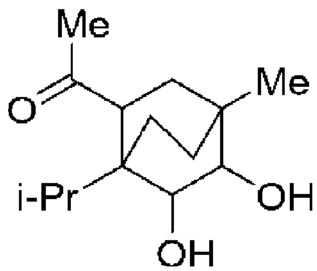

(Compound 21)

First, 7 g of the mixture of compound 18 and compound 19, 132 ml of tert-butyl alcohol, and 33 ml of water were added to a 1000 ml three-neck flask equipped with a dropping funnel, and cooled to 0°C. Then, 7.91 g of potassium permanganate, 1.79 g of sodium hydroxide, and 165 ml of water were added to a different flask to prepare an aqueous permanganate solution which was then added to the dropping funnel. Potassium permanganate was slowly added dropwise such that the internal temperature did not exceed 5°C. After dropwise addition was complete, stirring was continued for 1 hour, and a saturated aqueous sodium thiosulfate solution was added dropwise until the reddish purple color of the aqueous layer disappeared. Then, 350 ml of ethyl acetate was added, and the mixture was left to stand still. After the organic layer of the supernatant was extracted, 350 ml of ethyl acetate was again added to the aqueous layer, and the mixture was left to stand still. After the organic layer of the supernatant was extracted and combined with the previous organic layer, the organic layer was washed with brine, dried over magnesium sulfate, and concentrated in a rotary evaporator to give a crude product. The crude product was purified by silica gel column chromatography to give 2.1 g of a mixture of a compound 20 and a compound 21 as an isomeric mixture.

<Synthesis of compound 22 and compound 23>

Compounds 22 and 23 shown below were synthesized by the method described below.

[Formula 33]

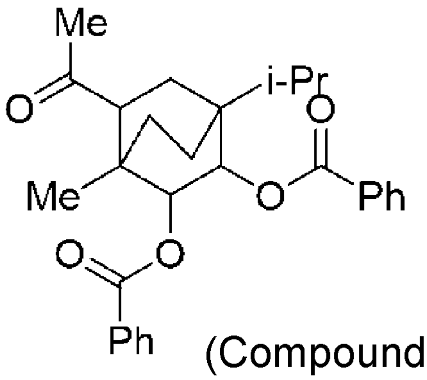
(Compound 22)

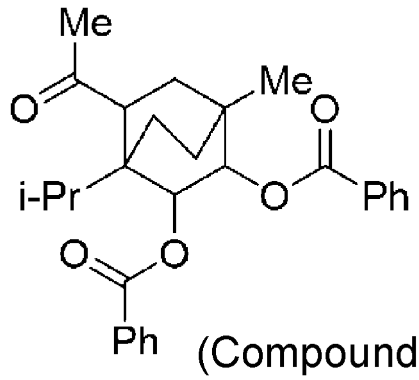
(Compound 23)

In a nitrogen atmosphere, 1.93 g of the mixture of compound 20 and compound 21 and 8 ml of dehydrated pyridine were added to a 50 ml three-neck flask. After the mixture was cooled in an ice bath, 2 ml of benzoyl chloride was slowly added dropwise, the temperature was raised to room temperature, and stirring was continued overnight. Dichloromethane and water were added to the reaction solution to separate the organic layer, and the aqueous layer was extracted three times with dichloromethane. The organic layer was washed with brine, dried over magnesium sulfate, and then concentrated in a rotary evaporator. The reaction mixture was purified by silica gel column chromatography to give 2.34 g of a mixture of a compound 22 and a compound 23 (an isomeric mixture mixed in a ratio of 71:29).

$^{1}$H NMR (270 MHz, $CDCl_3$, TMS as an internal standard): δ 7.87-7.71 (m, 4H), 7.50-7.27 (overlapping with signal of $CHCl_3$, m, 4H), 7.15-7.09 (m, 2H), 6.20 (dd, J = 7.6 Hz, 1.3 Hz, 1H, major isomer), 5.79 (dd, J = 7.9 Hz, 2.3 Hz, 1H, minor isomer), 5.65-5.63 (m, 1H, minor isomer), 5.49 (dd, J = 7.9 Hz, 2.0 Hz, 1H, major isomer), 3.02 (dd, J = 11.9 Hz, 4.9 Hz, 1H, major isomer), 2.81 (t, J = 9.2 Hz, 1H, minor isomer), 2.27 (s, 3H, major isomer), 2.26 (s, 3H, minor isomer), 2.17-1.23 (m, 7H), 0.94-0.81 (m, 9H)

[Example 13] (Synthesis of ester compound)

<Synthesis of compound 24 and compound 25>

Compounds 24 and 25 shown below were synthesized by the method described below.

[Formula 34]

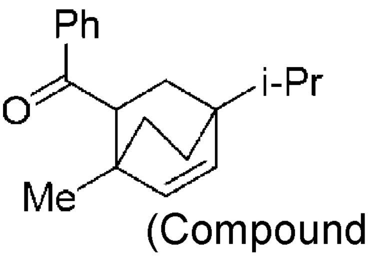
(Compound 24)

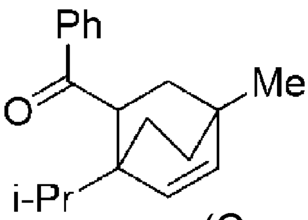
(Compound 25)

In a nitrogen atmosphere, 2.0 g of anhydrous aluminum chloride and 18 ml of dehydrated toluene were added to a 50 ml two-neck flask and stirred at room temperature. An aluminum chloride solution was prepared by slowly adding 2.3 ml of dehydrated THF dropwise to dissolve aluminum chloride. In a nitrogen atmosphere, 9.4 g of phenyl vinyl ketone, 11.6 g of α-terpinene, and 40 ml of dehydrated toluene were added to a 300 ml three-neck flask and stirred while being cooled in an ice bath. The previously prepared aluminum chloride solution was slowly added dropwise to a 300 ml flask and then heated to room temperature, and the mixture was stirred overnight. The mixture was cooled in an ice bath again, and 150 ml of water was added to terminate the reaction. The mixture was separated into an organic layer and an aqueous layer, the organic layer was washed with 100 ml of water, a saturated aqueous sodium bicarbonate solution, and brine in this order, and the organic layer was dried over magnesium sulfate and then concentrated in a rotary

evaporator. The resulting crude product was purified by silica gel column chromatography to give 10.9 g of an isomeric mixture of a compound 24 and a compound 25.

<Synthesis of compound 26 and compound 27>

Compounds 26 and 27 shown below were synthesized by the method described below.

[Formula 35]

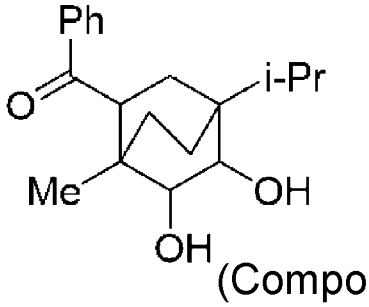
(Compound 26)

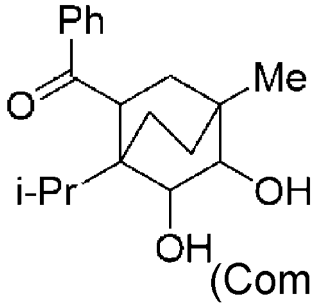
(Compound 27)

First, 10.8 g of the mixture of compound 24 and compound 25, 200 ml of tert-butyl alcohol, and 40 ml of water were added to a 1000 ml three-neck flask equipped with a dropping funnel, and cooled to 0°C. Then, 6.98 g of potassium permanganate, 1.93 g of sodium hydroxide, and 160 ml of water were added to a different flask to prepare an aqueous permanganate solution which was then added to the dropping funnel. Potassium permanganate was slowly added dropwise such that the internal temperature did not exceed 6°C. After dropwise addition was complete, stirring was continued for 1 hour, and a saturated aqueous sodium thiosulfate solution was added dropwise until the reddish purple color of the aqueous layer disappeared. Then, 350 ml of ethyl acetate was added, and the mixture was stirred and left to stand still. After the organic layer of the supernatant was extracted, 350 ml of ethyl acetate was again added to the aqueous layer, and the mixture was stirred and left to stand still. After the organic layer of the supernatant was extracted and combined with the previous organic layer, the organic layer was washed with brine, dried over magnesium sulfate, and concentrated in a rotary evaporator to give a crude product. The crude product was purified by silica gel column chromatography to give 4.0 g of an isomeric mixture of a compound 26 and a compound 27.

<Synthesis of compound 28 and compound 29>

Compounds 28 and 29 shown below were synthesized by the method described below.

[Formula 36]

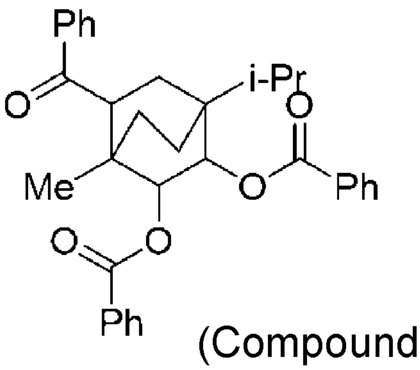
(Compound 28)

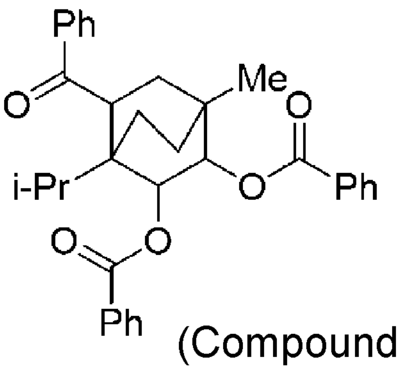
(Compound 29)

In a nitrogen atmosphere, 3.6 g of the mixture of compound 26 and compound 27 and 5 ml of dehydrated

pyridine were added to a 50 ml three-neck flask. After the mixture was cooled in an ice bath, 2.9 ml of benzoyl chloride was slowly added dropwise, the temperature was raised to room temperature, and stirring was continued overnight. Dichloromethane and water were added to the reaction solution to separate the organic layer, and the aqueous layer was extracted three times with dichloromethane. The organic layer was washed with brine, dried over magnesium sulfate, and then concentrated in a rotary evaporator. The reaction mixture was purified by silica gel column chromatography to give 3.42 g of a mixture of a compound 28 and a compound 29 (an isomeric mixture mixed in a ratio of 76:24).

$^1H$ NMR (270 MHz, $CDCl_3$, TMS as an internal standard): δ 8.03-7.09 (m, 15H), 6.51 (dd, J = 7.6 Hz, 1.3 Hz, 1H, major isomer), 6.08 (dd, J = 7.9 Hz, 1.6 Hz, 1H, minor isomer), 5.74 (dd, J = 7.9 Hz, 1.0 Hz, 1H, minor isomer), 5.70 (dd, J = 7.6 Hz, 2.0 Hz, 1H, major isomer), 3.84 (dd, J = 11.9 Hz, 4.9 Hz, 1H, major isomer), 3.70 (t, J = 9.2 Hz, 1H, minor isomer), 2.25-1.23 (m, 7H), 0.93 - 0.75 (m, 9H)

The melting point of the resulting mixture of compound 28 and compound 29 was measured with a differential scanning calorimeter (DSC 7020 manufactured by Hitachi High-Tech Science Corporation, start temperature: 25°C, end temperature: 300°C, heating rate: 10°C/min) and was 130°C.

[Example 14]

<Preparation of solid titanium catalyst component [α1]>

After a 1-liter glass vessel was sufficiently purged with nitrogen, 85.8 g of anhydrous magnesium chloride, 321 g of decane, and 352 g of 2-ethylhexyl alcohol were placed therein, and thermally reacted at 130°C for 3 hours to give a homogeneous solution. Then, 241 g of this solution and 6.43 g of ethyl benzoate were added to the glass vessel, and mixed by being stirred at 50°C for 1 hour.

The homogeneous solution thus obtained was cooled to room temperature. Then, 38.3 mL of this entire homogeneous solution was added dropwise into 100 mL of titanium tetrachloride retained at -20°C under stirring at a speed of 350 rpm for 45 minutes. After the addition was finished, the temperature of this mixture was raised to 80°C over 3.8 hours. When the temperature reached 80°C, 1.26 g of the compound 10 was added into the mixture. The temperature was raised again to 120°C over 40 minutes, and the same temperature was retained under stirring for 35 minutes. After the reaction finished, the solid portion was recovered by hot filtration. This solid portion was resuspended in 100 mL of titanium tetrachloride, and then the suspension was subjected to a heating reaction again at 120°C for 35 minutes. After the reaction finished, the solid portion was recovered again by hot filtration and sufficiently washed with decane at 100°C and decane at room temperature until no free titanium compound was detected in the decane solution after washing the solid. A solid titanium catalyst component [α1] containing the compound 10 and prepared by the procedure above was preserved as a decane slurry, and a portion thereof was dried in order to examine the catalyst composition. The composition of the solid titanium catalyst component [α1] thus obtained included 0.28% by mass titanium, 1.5% by mass magnesium, and 0.13% by mass 2-ethylhexyl alcohol residues.

<Main polymerization>

After the addition of 500 g of propylene and 1 NL of hydrogen at room temperature to a two-liter autoclave, the mixture which was obtained by mixing 7 mL of heptane, 0.35 mmol of triethyl aluminum, 0.07 mmol of cyclohexylmethyldimethoxysilane, and 0.0028 mmol of solid titanium catalyst component [α1] (in terms of titanium atom) at 25°C for 10 minutes was added thereto, and the temperature inside the autoclave was raised quickly to 70°C. After polymerization at 70°C for 1.5 hours, the reaction was stopped by the addition of a small amount of methanol, and then propylene was purged. Finally, the obtained polymer particles were dried under reduced pressure at 80°C overnight. Table 1 shows, for example, activity, bulk specific gravity, MFR, amount of decane-insoluble component, Tm, Tmf, ΔH, and MWD (a molecular weight distribution).

[Table 1]

Table 1

| | Activity (Kg-PP/g-Cat) | Bulk specific gravity (g/ml) | MFR (g/10 min) | Content of decane-soluble component (%) |
|---|---|---|---|---|
| Example 14 | 57.1 | 0.48 | 4.4 | 1.3 |

(continued)

| Table 1 (continued) | | | | | | | |
|---|---|---|---|---|---|---|---|
| | Mw | Mw/Mn | Mz/Mw | Tmf (°C) | Tm (°C) | Tc (°C) | ΔH (J/g) |
| Example 14 | 424000 | 8.86 | 7.41 | 171.3 | 161.9 | 113.5 | 85.8 |

Methods for measuring the physical properties described above are as follows.

(1) Bulk specific gravity:
The bulk specific gravity was measured in accordance with JIS K-6721.

(2) Melt flow rate (MFR):
In accordance with ASTM D1238E, the measurement temperature for a propylene polymer was set at 230°C.

(3) Amount of decane-soluble (insoluble) component:
To a glass measurement vessel, about 3 g of propylene polymer (It was measured to the unit of $10^{-4}$ g. This weight was denoted as b (g) in the following equation.), 500 mL of decane, and a small amount of a heat-resistant stabilizer soluble in decane were added. Under a nitrogen atmosphere, the temperature was raised to 150°C over 2 hours under stirring with a stirrer to dissolve the propylene polymer, the temperature was retained at 150°C for 2 hours, and then the solution was gradually cooled to 23°C over 8 hours. The liquid containing the precipitate of the propylene polymer obtained was filtered under reduced pressure with a 25G-4 standard glass filter manufactured by Iwata Garasu Corporation. 100 mL of the filtrate was harvested and dried under reduced pressure to give a portion of a decane soluble component. The weight thereof was weighed to the $10^{-4}$ g unit (this weight was denoted as a(g) in the following equation). After this operation, the amount of the decane-soluble component was determined by the following equation.

$$\text{Decane-soluble component content} = 100 \times (500 \times a) / (100 \times b)$$

$$\text{Decane-insoluble component content} = 100 - 100 \times (500 \times a) / (100 \times b)$$

(4) Molecular weight distribution:

Gel permeation chromatograph: HLC-8321 GPC/HT model manufactured by Tosoh Corporation
Detector: Differential refractometer
Column: TSKgel GMH6-HT × 2 and TSKgel GMH6-HTL × 2 manufactured by Tosoh Corporation were serially connected.
Mobile phase medium: o-dichlorobenzene
Flow rate: 1.0 ml/min
Measurement temperature: 140°C
Method of creating calibration curve: a standard polystyrene sample was used.
Sample concentration: 0.1% (w/w)
Amount of sample solution: 0.4 ml

Measurement was conducted under the conditions described above. The chromatogram obtained was analyzed by a known method to calculate the weight average molecular weight (Mw), the number average molecular weight (Mn), the Z average molecular weight (Mz), and the Mw/Mn value and Mz/Mw value as indices for the molecular weight distribution (MWD). The measurement time for one sample was 60 minutes.
(5) Melting point (Tm) of polymer:

The melting point (Tm), crystallization temperature (Tc), and amount of heat of fusion (ΔH) of the polymer in the present invention were measured by a differential scanning calorimeter (DSC) in a DSC220C apparatus manufactured by Seiko Instruments Inc. 3 to 10 mg of a specimen was sealed in an aluminum pan and heated from room temperature to 200°C at 100°C/minute. The specimen was retained at 200°C for 5 minutes and then cooled to 30°C at 10°C/minute. The peak temperature in this cooling test was defined as the crystallization temperature (Tc). Subsequently, after retained at 30°C for 5 minutes, the specimen was subjected to a second heating to 200°C at 10°C/minute. In this second heating test, the peak temperature was employed as the melting point (Tm), and the heat generation quantity was employed as the amount of heat of fusion (ΔH).

The final melting point (Tmf) of the polymer in the present invention was measured by a differential scanning calorimeter (DSC) in a DSC220C apparatus manufactured by Seiko Instruments Inc. 3 to 10 mg of a specimen was sealed in an aluminum pan and heated from room temperature to 240°C at 80°C/minute. The specimen was retained at 240°C for 1 minute and then cooled to 0°C at 80°C/minute. After retained at 0°C for 1 minute, the specimen was heated to 150°C at 80°C/minute and retained for 5 minutes. Finally, the specimen was heated to 180°C at 1.35°C/minute, and the intersection of the tangent of the inflection point on the high-temperature side of the peak provided in this final heating test and the base line was employed as the final melting point (Tmf).

Tmf can be considered as one parameter for evaluating, for example, the ease of crystallization and the strength of the crystal structure of a polymer in the ultra-high molecular weight region, which is said to tend to be difficult to crystallize, and the strength of the crystal structure of a component having extremely high crystallinity. More specifically, it can be considered that, as this Tmf value is higher, the ultra-high molecular weight polymer component is more likely to form crystals that is strong and has high heat resistance.

[Example 15] (Synthesis of ester compound)

<Synthesis of compound 30>

A compound 30 shown below was synthesized by the method described below.

[Formula 37]

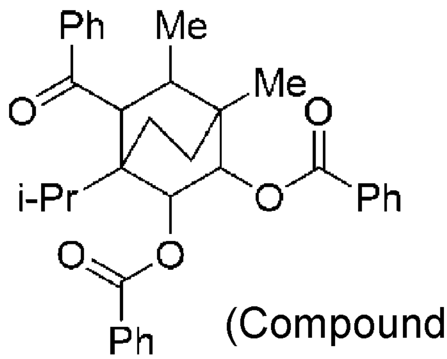

(Compound 30)

(Synthesis of precursor (cyclic olefin compound))

In a nitrogen atmosphere, 4.5 g of anhydrous aluminum chloride and 90 ml of dehydrated toluene were added to a 200 ml two-neck flask and stirred at room temperature. An aluminum chloride solution was prepared by slowly adding 5.6 ml of dehydrated THF dropwise to dissolve aluminum chloride. In a nitrogen atmosphere, 24.3 g of phenyl-1-propenyl ketone, 28.0 g of α-terpinene, and 170 ml of dehydrated toluene were added to a 500 ml three-neck flask and stirred while being cooled in an ice bath. The previously prepared aluminum chloride solution was slowly added dropwise to a 500 ml flask and then heated to room temperature, and the mixture was stirred overnight. The mixture was cooled in an ice bath again, and 150 ml of water was added to terminate the reaction. The reaction solution was added to a separatory funnel, and then 300 ml of methylene chloride was added. After the organic layer and the aqueous layer were separated, the organic layer was washed with 300 ml of water and separated again. The organic layer was washed with a saturated aqueous sodium bicarbonate solution and brine, dried over magnesium sulfate, and then concentrated in a rotary evaporator. The resulting crude product was purified by silica gel column chromatography to give 12.0 g of a Diels-Alder adduct (a cyclic olefin compound).

(Synthesis of diol compound)

First, a 1000 ml three-necked flask was charged with 12.0 g of the Diels-Alder adduct, 167 ml of tert-butanol, and 42 ml of water, then equipped with a dropping funnel, and cooled to 0°C. Then, 10.0 g of potassium permanganate, 2.2 g of sodium hydroxide, and 209 ml of water were added to a different flask to prepare an aqueous permanganate solution which was then added to the dropping funnel. Potassium permanganate was slowly added dropwise such that the internal temperature did not exceed 5°C. After dropwise addition was complete, stirring was continued for 1 hour, and a saturated aqueous sodium thiosulfate solution was added dropwise until the reddish purple color of the aqueous layer disappeared. After 350 ml of ethyl acetate was added and the mixture was stirred, the supernatant was left to stand still. After the organic layer of the supernatant was extracted, 350 ml of ethyl acetate was added to the aqueous layer, the mixture was stirred, and then the supernatant was again left to stand still. The organic layer of the supernatant was extracted in the same manner, and combined with the aforementioned organic layer. The organic layer was washed with brine, dried over magnesium sulfate, and then concentrated in a rotary evaporator to give 14.0 g of a crude product. The crude product was purified by silica gel column chromatography to give 3.84 g of diol compound.

(Synthesis of ester compound 30)

In a nitrogen atmosphere, 3.84 g of the above diol and 12.4 ml of dehydrated pyridine were added to a 50 ml three-neck flask. After the mixture was cooled in an ice bath, 2.5 ml of benzoyl chloride was slowly added dropwise, the temperature was raised to room temperature, and stirring was continued overnight. Dichloromethane and water were added to the reaction solution to separate the organic layer, and the aqueous layer was extracted three times with dichloromethane. The organic layer was washed with brine, dried over magnesium sulfate, and then concentrated in a rotary evaporator. The reaction mixture was purified by silica gel column chromatography to give 2.11 g of a compound 30 as a main product.

$^1H$ NMR (270 MHz, $CDCl_3$, TMS as an internal standard): $\delta$ 8.08-8.01 (m, 2H), 7.91-7.83 (m, 2H), 7.82-7.72 (m, 2H), 7.65-7.23 (m, 7H), 7.20-7.07 (m, 2H), 6.38 (d, J = 7.6 Hz, 1H), 5.72-5.61 (m, 1H), 3.42 (d, J = 5.3 Hz, 1H), 2.16-1.93 (m, 2H), 1.86-1.44 (m, 4H), 1.12 (d, J = 6.9 Hz, 3H), 0.87-0.69 (m, 9H)

The melting point of the resulting compound 30 was measured with a differential scanning calorimeter (DSC 7020 manufactured by Hitachi High-Tech Science Corporation, start temperature: 25°C, end temperature: 300°C, heating rate: 10°C/min) and was 60°C.

**Industrial Applicability**

The novel ester compound according to the present invention is a compound useful for production of resin additives, cosmetics materials and external preparations for skin, microbicidal compositions, antioxidants, chelators, and Ziegler-Natta catalysts. The compound can be utilized particularly as a catalyst component for Ziegler-Natta catalysts, and enables production of a catalyst that imparts excellent stereoregularity and productivity on polymerization of polypropylene. As described above, the ester compound of the present invention has an extremely high industrial value.

**Claims**

1. A cyclic multiple-ester-group-containing compound (A) represented by the following formula (1):

[Formula 1]

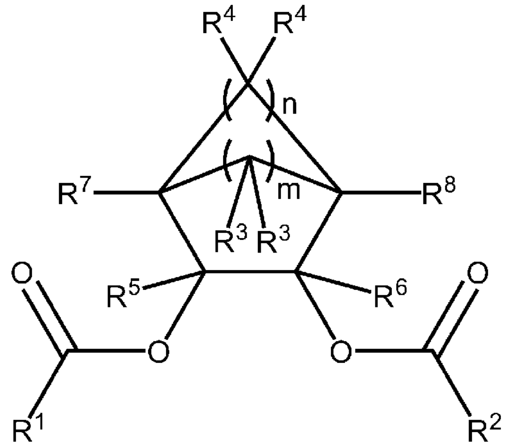

(1)

wherein m and n are each independently an integer of 1 to 5, with a relationship of $m+n \geq 4$ being satisfied; $R^1$ and $R^2$ are each independently a substituted or unsubstituted hydrocarbon group having 1 to 20 carbon atoms; a plurality of $R^3$, a plurality of $R^4$, and $R^5$ to $R^8$ are each independently a group selected from a hydrogen atom, a substituted or unsubstituted hydrocarbon group having 1 to 20 carbon atoms, or a halogen atom; a hydrogen atom, a carbon atom, or both, of $R^1$ to $R^8$ are optionally replaced by at least one atom selected from the group consisting of a nitrogen atom, an oxygen atom, a phosphorus atom, a halogen atom, and a silicon atom; while $R^3$ to $R^8$ are in a mutually independent relationship, adjacent $R^3$ groups are optionally directly bonded to one another to form a multiple bond; adjacent $R^4$ groups are optionally directly bonded to one another to form a multiple bond; and a plurality of $R^3$ bonded to the same carbon, and a plurality of $R^4$ bonded to the same carbon, are optionally bonded to one another to form a cyclic structure.

2. The ester compound (A) according to claim 1, wherein m is 2 or more, and n is 2 or more.

3. The ester compound (A) according to claim 1, wherein $R^1$ and $R^2$ are each independently a substituted or unsubstituted alkyl group, a substituted or unsubstituted alkenyl group, a substituted or unsubstituted cycloalkyl group, a substituted or unsubstituted aryl group, or a substituted or unsubstituted heteroaryl group.

4. The ester compound (A) according to claim 1, wherein $R^3$ to $R^8$ are each independently a group selected from a hydrogen atom, a substituted or unsubstituted alkyl group, a substituted or unsubstituted alkenyl group, a substituted or unsubstituted cycloalkyl group, a substituted or unsubstituted cycloalkenyl group, a substituted or unsubstituted alkoxy group, a substituted or unsubstituted alkenyloxy group, a substituted or unsubstituted cycloalkyloxy group, a substituted or unsubstituted cycloalkenyloxy group, a substituted or unsubstituted aryl group, a substituted or unsubstituted aryloxy group, a substituted or unsubstituted heteroaryl group, or a substituted or unsubstituted heteroaryloxy group.

**INTERNATIONAL SEARCH REPORT**

International application No.
**PCT/JP2021/047303**

**A. CLASSIFICATION OF SUBJECT MATTER**

***C07C 69/76***(2006.01)i; ***C07C 69/78***(2006.01)i; ***C07C 69/92***(2006.01)i; *C08F 10/06*(2006.01)n
FI: C07C69/76 A; C07C69/76 Z; C07C69/78 CSP; C07C69/92; C08F10/06

According to International Patent Classification (IPC) or to both national classification and IPC

**B. FIELDS SEARCHED**

Minimum documentation searched (classification system followed by classification symbols)

C07C69/76; C07C69/78; C07C69/92; C08F10/06

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | CN 106892820 A (XIU JIANDONG) 27 June 2017 (2017-06-27) claims, paragraph [0005] | 1-4 |
| X | NAEMURA, K et al., Pig Liver Esterase-catalyzed Hydrolyses of Racemic Diacetates of Bicyclic Compounds and Interpretation of the Enantiomeric Specificity of PLE, CHEMISTRY LETTERS, 1991, vol. 20, pp. 657-660 table 1 | 1-4 |
| X | BURDISSO, M et al., Diastereofacial Selectivity in 1,3-Dipolar Cycloadditions. Reactions of Diazomethane with endo,cis-5,6-Disubstituted Bicyclo[2.2.2]oct-2-enes., Tetrahedron, 1991, vol. 47, pp. 7699-7712 scheme 1 | 1-4 |
| X | BURDISSO, M et al., ROLE OF STERIC, ELECTROSTATIC AND HYDROGEN BONDING EFFECTS AS FACE SELECTIVITY CONTROLLING FACTORS IN NITRONE CYCLOADDITIONS, Tetrohedron Letters, 1987, vol. 28, pp. 1225-1228 p. 1227 | 1-4 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 February 2022** | **15 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2021/047303

C. DOCUMENTS CONSIDERED TO BE RELEVANT

| Category* | Citation of document, with indication, where appropriate, of the relevant passages | Relevant to claim No. |
|---|---|---|
| X<br>A | WATANABE, A et al., Synthetic study of optically active and C2-symmetric novel ligand, 7,8-bis(benzyloxy)bicyclo[2.2.2]octa-2,5-diene and aq tricarbonyliron complex, Organic & Biomolecular Chemistry, 2003, vol. 1, pp. 1726-1729<br>scheme 1 | 1-3<br>4 |
| X<br>A | COTTERILL, IC et al., Diels-Alder Reactions involving cis-1,2-Isopropylidenedioxycyclohexa-3,5-diene and Enzymatic Resolution of One of the Adducts, Journal of the Chemical Society, Chemical Communications, 1988, vol. 24, pp. 1628-1629<br>p. 1629 | 1-3<br>4 |
| X<br>A | BARAN, A et al., Synthesis of Bicyclo[2.2.2]octane-2,3,5,6,7,8 hexols (Bishomoinositols) as Glycosidase Inhibitors, The Journal of Organic Chemistry, 2008, vol. 73, pp. 4370-4375<br>schemes 1-4 | 1-3<br>4 |
| X<br>A | OGAWA, S et al., Total Synthesis and Absolute Configuration of Zeylena, The Journal of Organic Chemistry, 1985, vol. 50, pp. 5075-5077<br>p. 5076 | 1-3<br>4 |
| X | CAS REGISTRY NO. 2059675-44-2, DATABASE REGISTRY, [online], 26 January 2017, [retrieved date 28 January 2022], Retrieved from: STN<br>entire text | 1-4 |
| A | CN 108250335 A (YINGKOU FENGGUANG ADVANCED MATERIAL CO., LTD.) 06 July 2018 (2018-07-06)<br>claims, paragraphs [0012]-[0016], examples | 1-4 |
| A | JP 10-7716 A (MITSUI CHEMICALS, INC.) 13 January 1998 (1998-01-13)<br>claims, paragraphs [0031]-[0036], examples | 1-4 |

Form PCT/ISA/210 (second sheet) (January 2015)

**INTERNATIONAL SEARCH REPORT**
**Information on patent family members**

International application No.
**PCT/JP2021/047303**

| Patent document cited in search report | Publication date (day/month/year) | Patent family member(s) | Publication date (day/month/year) |
|---|---|---|---|
| CN 106892820 A | 27 June 2017 | (Family: none) | |
| CN 108250335 A | 06 July 2018 | (Family: none) | |
| JP 10-7716 A | 13 January 1998 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005226076 A
- JP 2000516987 A
- JP 2002542347 A
- JP 2005517746 A
- JP 2011529888 A
- JP 2014500390 A
- JP 2008247796 A
- WO 2008062553 A
- US 20180149973 A1
- US 20020162991 A1


### Non-patent literature cited in the description


- *Journal of Organic Chemistry,* 1969, vol. 34, 3579-3582
- *Journal of Organic Chemistry,* 1971, vol. 36, 3979-3987
- *Organic Letters,* 2004, vol. 6, 1589-1592
- *Journal of the American Chemical Society,* 1957, vol. 79, 2822-2824
- *Organic Synthesis,* 1991, vol. 70, 47-53
- *Organic Synthesis,* 1997, vol. 75, 153-160
- *Catalysis Letters,* 2012, vol. 142, 124-130
- *Organic Synthesis,* 1997, vol. 74, 91-100
- Synthesis of Organic Compound II, Alcohols and amines. *The Fourth Series of Experimental Chemistry,* vol. 20, 39
- *Journal of Organic Chemistry,* 1959, vol. 24, 54-55
- *Angewandte Chemie International Edition,* 1978, vol. 17, 522-524
- *Bulletin of the Chemical Society of Japan,* 1967, vol. 40, 2380-2382
- *Organic Synthesis,* 1952, vol. 32, 41
- *Macromolecules,* 2017, vol. 50, 580-586
- *Journal of Organic Chemistry,* 1980, vol. 45, 2301-2304
- *Journal of Organic Chemistry,* 2009, vol. 74, 405-407
- *Journal of Organic Chemistry,* 1988, vol. 53, 2120-2122
- *Journal of Organic Chemistry,* 1963, vol. 28, 2572-2577
- *European Journal of Organic Chemistry,* 2017, vol. 24, 3501-3504